(19) Europäisches Patentamt
European Patent Office
Office européen des brevets

(11) Publication number: **0 187 297 B1**

(12) # EUROPEAN PATENT SPECIFICATION

(45) Date of publication of patent specification: **21.08.91**

(51) Int. Cl.5: **C07C 69/30**, C07C 67/08, C07C 69/76, C07C 67/30, C07D 473/18, C07C 69/14

(21) Application number: **85115803.0**

(22) Date of filing: **11.12.85**

(54) **Alkoxy methyl ether and alkoxy methyl ester derivatives.**

(30) Priority: **12.12.84 US 681037**
**12.11.85 US 795381**

(43) Date of publication of application:
**16.07.86 Bulletin 86/29**

(45) Publication of the grant of the patent:
**21.08.91 Bulletin 91/34**

(84) Designated Contracting States:
**AT BE CH DE FR GB IT LI LU NL SE**

(56) References cited:
**EP-A- 0 018 342**
**EP-A- 0 074 306**
**EP-A- 0 085 424**
**EP-A- 0 145 207**

**CHEMICAL ABSTRACTS, vol. 68, no. 3, 15 Jan 1968, Columbus, OH (US); S.MAMADOV et al.: "Ethers of glycols and their derivatives. CII. Synthesis of alkoxymethyl ethers from 1,3-dialkoxy-2-propanol", p. 1166, no. 12384v**

(73) Proprietor: **SYNTEX (U.S.A.) INC.**
**3401 Hillview Avenue**
**Palo Alto California 94303(US)**

(72) Inventor: **Morgans, David John, Jr**
**651 Melville Avenue**
**Palo Alto, CA 94301(US)**
Inventor: **Chapman, Harlan Hanford**
**805 Colorado Avenue**
**Palo Alto, CA 94303(US)**

(74) Representative: **Barz, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to alkoxymethyl ethers and alkoxymethyl esters of glycerols and to methods for producing alkoxymethyl ethers and alkoxymethyl esters of glycerols. In particular this invention relates to intermediates and methods for producing intermediates for the production of 9-(1,3-dihydroxy-2-propoxymethyl) guanine and its esters and ethers.

9-(1,3-dihydroxy-2-propoxymethyl) guanine (hereinafter DHPG) and its esters are potent antiviral agents and have been prepared by methods disclosed in US-A-4 355 032 and EP-A-49 072, 72 027, 74 306 and 85 424. Similar compounds having similar side chains are disclosed in US-A-4 347 360 and 4 199 574. The present invention relates to new intermediates and to an improved process whereby the new DHPG ether or ester side chain intermediate is prepared in fewer steps than other currently used processes and produces fewer undesirable by-products.

The side chain intermediates for the preparation of DHPG and its ethers and esters have been produced in the past by reacting epichlorohydrin with a benzyl alcohol, to obtain a symmetrically substituted 1,3-dibenzyl glycerol, which is then chloromethylated. The chloromethyl compound is converted to the acetate or formate ester, which is then reacted with guanine to add to the 9 position of guanine. See US-A-4 355 032.

An alternate synthesis is disclosed in US-A-4 347 360 where 1,3-dichloro-2 propanol is reacted with sodium benzylate forming the chloromethoxy derivative which is reacted further with a purine base.

A third method is disclosed in EP-A-74 306. In this application the side chain is made by starting with glycerol formal, a mixture of 1,3-dioxan-5-ol and 1,3-dioxolane-4-methanol. The glycerol formal is acylated and the mixture is separated. The acylated compound is then reacted with acetic anhydride in the presence of $ZnCl_2$ to give a compound that can be reacted with a purine.

These syntheses are all multistep methods that involve complicated separation and purification techniques and do not involve the intermediates of this invention.

It would be advantageous to have a process that was straight forward (required fewer steps), used easily obtainable starting materials, did not create unwanted isomers, and allowed facile substitution of the terminal esters. The claimed invention provides all these features. In particular, DHPG and a wide variety of 1,3-di- esters and 1,3-di- ethers of DHPG can easily be made using the intermediates and process of this invention.

This invention provides a process for preparing a compound represented by the formula

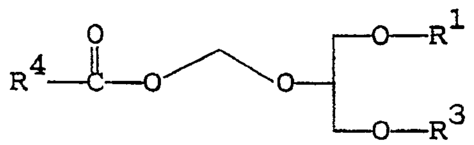

A

wherein $R^1$ and $R^3$ are selected from hydrogen, optionally substituted lower alkyl, acyl, 1-adamantanoyl, optionally substituted phenyl or optionally substituted phenyl lower alkyl and $R^4$ is optionally substituted lower alkyl, comprising:

a) reacting a compound of formula D

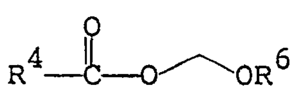

D

wherein $R^4$ is as defined above and $R^6$ is optionally substituted lower alkyl, with a compound of formula E

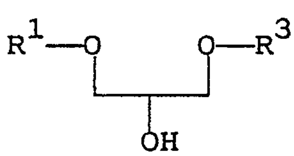

E

wherein $R^1$ and $R^3$ are as defined above, in the presence of a protic acid without external heat, to form a compound of formula C

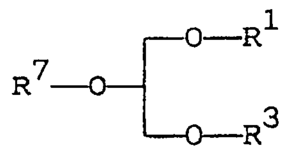

C

wherein $R^1$ and $R^3$ are as defined above and $R^7$ is $R^4C(O)OCH_2$ or $R^6OCH_2$ wherein $R^4$ and $R^6$ are as defined above;

b) optionally followed by, if $R^7$ is $R^6OCH_2$, reacting a compound represented by formula B

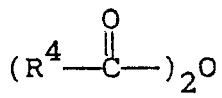

B

wherein $R^4$ is independently selected from the group as defined above, with a compound of formula C in which $R^7$ is $R^6OCH_2$, in the presence of a Lewis acid for about 60 to 180 minutes at a temperature of between 45° and 65° C.

This invention further provides a method for making compounds represented by the formula

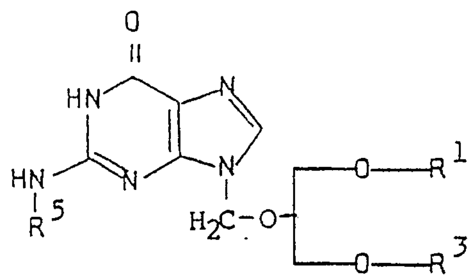

wherein $R^1$ and $R^3$ are selected from hydrogen, optionally substituted lower alkyl, acyl, 1-adamantanoyl, optionally substituted phenyl or optionally substituted phenyl lower alkyl and $R^5$ is optionally substituted acyl or hydrogen, comprising:

a) preparing a compound of formula A according to the procedure described above; followed by

b) reacting the thus prepared compound of formula A with optionally substituted guanine; optionally followed by

c) treating the resulting mixture with a lower alkyl alcohol; and/or

d) if $R^1$ and $R^3$ are not hydrogen, treating the resulting mixture with ammonium hydroxide and water.

Furthermore this invention provides a class of compounds useful as intermediates in the process of this invention represented by the Formula:

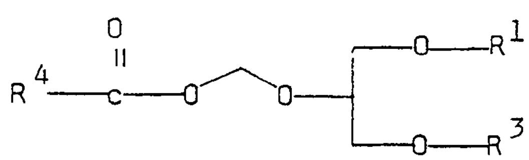

Formula A

wherein $R^1$ and $R^3$ are selected from hydrogen, optionally substituted lower alkyl, acyl, 1-adamantanoyl, optionally substituted phenyl or optionally substituted phenyl lower alkyl and $R^4$ is optionally substituted lower alkyl; except when $R^4$ is methyl, at least one of $R^1$ and $R^3$ is not acetyl, lower alkyl, optionally substituted phenyl or optionally substituted phenyl lower alkyl; and when $R^4$ is ethyl, $R^1$ and $R^3$ cannot both

be the same lower alkyl group.

Furthermore this invention provides a class of compounds useful as intermediates in the process of this invention represented by the Formula:

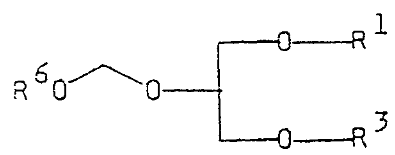

Formula F

wherein $R^1$, $R^3$ and $R^6$ are defined as optionally substituted lower alkyl, except that $R^1$ and $R^3$ cannot both be propyl.

As used in the specification and appended claims, unless specified to the contrary, the following terms have the meaning indicated.

"Lower alkyl" is defined as any straight or branched chain hydrocarbon group having 1 to 4 carbon atoms, for example, methyl, ethyl, propyl, i-propyl, butyl, t-butyl, and the like.

"Optionally substituted" refers to a substitution on an alkyl or phenyl group with halogen atoms, lower alkyl groups, and phenyl groups.

"Optionally substituted guanine" refers to a guanine molecule which is optionally substituted with acyl and/or $(alkyl)_3Si$ groups on the amino group at position 2 and on the nitrogen at position 9.

"Acyl" is defined herein as optionally substituted alkyl-C(O) having four to twenty carbon atoms in the alkyl chain, optionally substituted phenyl-C(O), or optionally substituted alkyl phenyl-C(O) where the optional substitution is on the phenyl ring. Examples include n-hexanoyl, n-heptanoyl, palmitoyl, stearyl, arachidoyl, pivaloyl, 1-methyl-1-cyclohexane-carboxoyl, 2-octyl-decanoyl, benzoyl, p-chlorobenzoyl, toluoyl, phenylacetyl, 3-phenylpropanoyl, and 4-phenylbutanoyl.

"Pivalic acid" is 2,2-dimethyl propionic acid.

"1-adamantanoyl" refers the radical having the structure identified below.

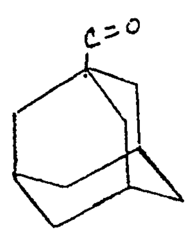

1-adamantanoyl

Phenyl refers to a six-member carbon ring which contains 3 double bonds.

Alkyl phenyl refers to a phenyl group which has an alkyl chain substituted on it wherein alkyl is as defined above.

"Hydrogen donor" refers to any substance that can serve as a source of active hydrogen. Some examples of hydrogen donors are hydrogen gas, cyclohexene, 1,4-cyclohexadiene and the like. In some cases catalysts may be used in conjunction with hydrogen donors, for example, the noble metals, for example, platinum, palladium, and rhodium may be used as a finely divided metal or as metal supported on any conventional catalyst support, for example, carbon, alumina, or silica.

"Acid" when used in this specification will normally be modified by either a specific named acid, for example, pivalic acid, or would be modified as a protic acid or a Lewis acid.

"Lewis acid" refers to any acid that does not have an abstractable proton. Examples include $Be(CH_3)_2$,

$BF_3$, $B(O\text{-alkyl})_3$, $Al(CH_3)$, $Al(Cl)_3$, $Fe^{3+}$,
$Mg^{2+}$, $Ca^{2+}$, $Cu^{+}$, $TiCl_4$, $Hg^{2+}$, $Co^{2+}$,
$Fe^{2+}$, $Zn^{2+}$,

and the like. A comprehensive list of Lewis acids can be found at Chem. Rev., 75, 1, (1975), on page 2.

"Protic acid" refers to any Bronsted acid having a $pK_a$ less than 2.0, and preferably less than 1.0 that has an abstractable proton. Examples include mineral acids, for example, nitric acid, sulfuric acid, phosphoric acid, hydrogen halides, for example, hydrochloric acid; organic acids, for example, trifloroacetic acid, organic sulfonic acids, for example phenyl sulfonic acid, and particularly preferred, para-toluene sulfonic acid; and acidic resins, for example Amberlyst® (Rohm and Haas).

"Protecting groups" refer to any group which can protect the functionality of a compound and may be removed by hydrolysis or hydrogenation. Examples of protecting groups useful in the present invention are sterically hindered groups (where sterically hindered refers to a particular atomic grouping which hinders or inhibits an expected chemical reaction due to its bulk), benzyl or optionally substituted benzyl, or alkyl groups, and sterically hindered silyl groups of the general formula $R_3Si(R_2)$-.

"Alkoxy methyl ether" means a compound represented by the formula

$A\text{-}O\text{-}CH_2\text{-}O\text{-}B$

where A and B are optionally substituted lower alkyl groups.

One process of the present invention is depicted in Reaction Scheme I.

## REACTION SCHEME I

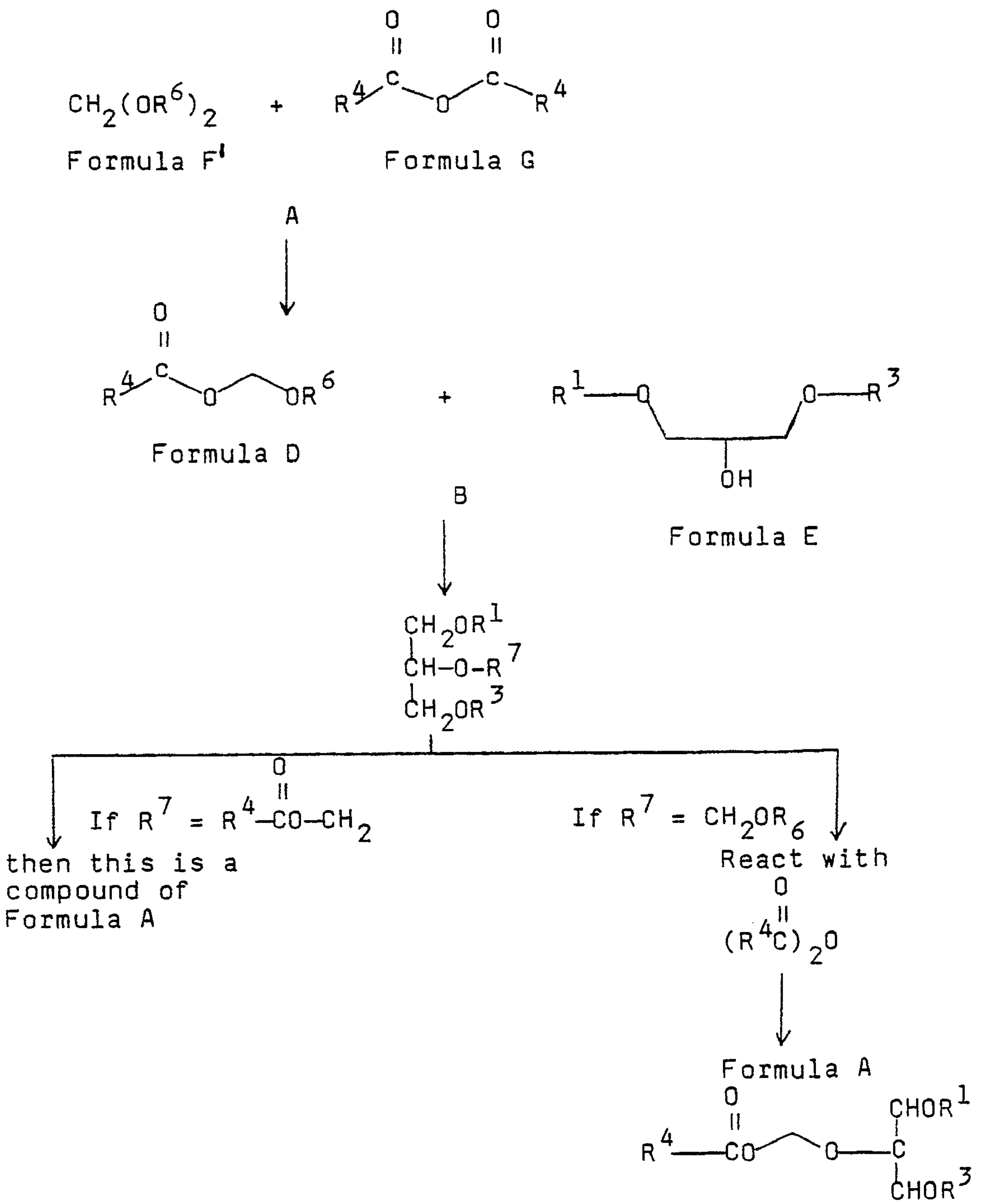


wherein $R^1$ and $R^3$ are selected from hydrogen, optionally subtituted lower alkyl, 1-adamantanoyl, acyl, optionally subtituted phenyl or optionally subtituted phenyl lower alkyl, and $R^7$ is $R^4C(O)OCH_2$ or $R^6OCH_2$ wherein $R^4$ and $R^6$ are independently selected from optionally subtituted lower alkyl.

In Reaction Sequence A a dialkyloxy methane Formula $F^1$ is reacted with an anhydride Formula G in the presence of a protic acid catalyst. The two reactants are present in about a one to one molar ratio in the presence of a catalytic amount of a protic acid, for example, organic sulfonic acids, for example, para-toluene sulfonic acid, or catalytic amounts of a mineral acid, for example, hydrochloric acid or sulfuric acid. The reaction can be done in an aprotic hydrocarbon solvent, for example benzene, or neat, typically at reflux temperature of the solution. The solution is reacted for 1 to 12 hours, 6 hours being about typical. See

the method of J. Am. Chem. Soc., 76, page 5161 (1954).

It is preferred that the anhydride and dialkyloxy methane be symmetric because the number of possible products is dramatically reduced by using symmetrical reagents. Asymmetrical anhydrides or asymmetrical alkyloxy methanes can, of course, be reacted together in the process of this invention. In certain cases asymmetrical anhydrides can be selective for one of the possible products.

The 1,3-dialkanoyl glycerol Formula E can be prepared in one of two ways. Treatment of glycerol with slightly more than two equivalents of an acid chloride or an acid anhydride in aprotic solvents in the presence of organic base, for example, trialkylamines, and a nucleophilic catalyst, for example, pyridine can afford the 1,3-dialkanoyl glycerol Formula E.

Alternatively these compounds are accessible from dihydroxyacetone using the method of J. Org. Chem., 35, 2082, (1970). Treatment of dihydroxyacetone in pyridine with an equivalent of the appropriate fatty acid chloride followed by reduction of the central keto group with borohydride in tetrahydrofuran can afford the 1,3-dialkanoyl glycerol Formula E.

In Reaction Sequence B compound Formula O and compound Formula E are reacted together in the presence of a protic acid catalyst, for example, organic sulfonic acids, for example, para-toluene sulfonic acid, or catalytic amounts of a mineral acid, for example, hydrochloric acid or sufuric acid, to produce a mixture (Formula C). This mixture is composed of compound of Formula A and compound of Formula F. Compounds of Formula D and Formula E are mixed together neat with a catalytic amount of a protic acid catalyst. It is preferable that an excess of the compound of Formula D be used, the excess being useful as a solvent for the reaction. The reactants can be mixed together without the necessity of an external heat source, although the application of heat will speed the reaction. Reaction B can be exothermic, and when the temperature drops to ambient temperature the reaction is over. The reaction takes between 1 and 6 hours, typically about 2 hours to complete.

The mixture (Formula C) will vary depending on the starting materials. For example, if $R^1$ and $R^3$ are benzyl, reaction sequence B produces mostly Formula A, but if $R^1$ and $R^3$ are pivaloyl reaction sequence B produces mostly Formula F. Reaction sequence C may be deleted if the product of Reaction Sequence B is a compound of Formula A. Otherwise, the compounds are reacted according to Reaction Sequence C.

In Reaction Sequence C, the mixture of compounds of Formula C is then further reacted in presence of an alkanoic anhydride and a Lewis acid catalyst. It is preferred that the anhydride used in this reaction be a symmetrical anhydride to reduce the possible number of products formed, although this is not a requirement for this reaction.

The catalytic acid is preferably a non-protic Lewis acid catalyst, for example, boron trifluoride, or aluminum trichloride. The reaction temperature climbs from ambient temperature to about 50 °C at which point temperature control is applied. The temperature of the reaction is generally kept between about 45° and 65 °C, preferably between about 50 °C and about 60 °C.

The progress of the reaction is typically followed by thin layer chromatography (TLC) to indicate the amount of reaction that has occurred. After between 60 and 180 minutes the reaction will normally be complete. The reaction mixture is extracted with an organic solvent which is washed with sodium carbonate and water. The organic layer is then dried and the product Formula A obtained by the evaporation of the solvent.

Once compound Formula A is obtained esters or ethers of DHPG or DHPG itself can then be easily obtained, if desired. Reaction Schemes II and III show this process.

REACTION SCHEME II

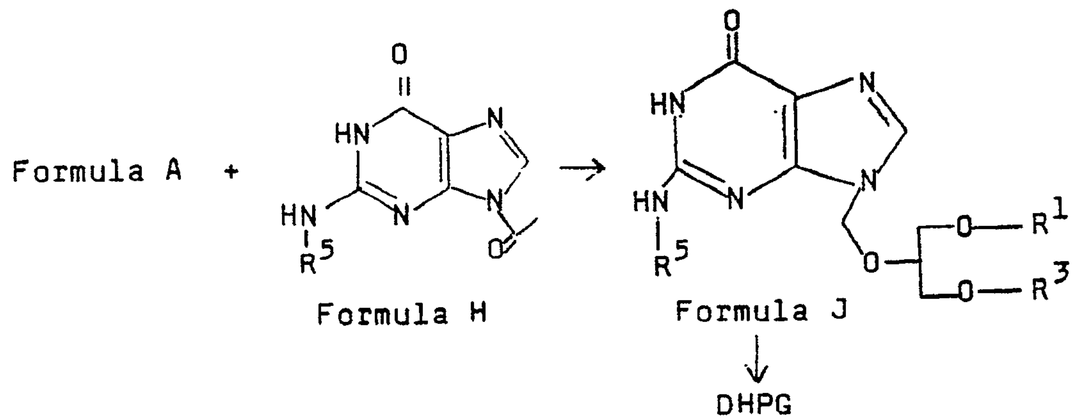


Compound Formula A can be converted into a 2-haloalkyl-1,3-diacyl glycerol using HX where X is a fluorine, chlorine, bromine or iodine atom, in methylene chloride.

Protected guanine Formula H produced for example by the methods of Tshido et al., Bull. Chem. Soc. Japan, 37, 1389 (1964), is reacted with compound Formula A, as isolated from Reaction Scheme I. $R^5$ is acyl or hydrogen and, if acyl, together with the guanine's nitrogen that $R^5$ protects, forms, preferably, a monoamide of a lower alkanoic acid, for example, acetamide, or propionamide. The reaction is conducted at elevated temperature under vacuum, in the presence of an acidic catalyst, for example para-toluene sulfonic acid. The DHPG derivative, Formula J, is then formed, where $R^1$ and $R^3$ are as previously defined. If the derivative ester or ether is directly useful, for example dipivaloyl DHPG or di-adamantanoyl DHPG, then no further reactions are necessary if $R^5$ was hydrogen. If $R^5$ was acyl, then the ester or ether of DHPG can be prepared by reacting compound of formula J with, for example, methanolic ammonia. If DHPG is desired, Formula J can be deprotected (removal of $R^1$, $R^3$, and $R^5$), forming DHPG, by reacting the compound with the mixture of, for example, ammonium hydroxide and water.

REACTION SCHEME III

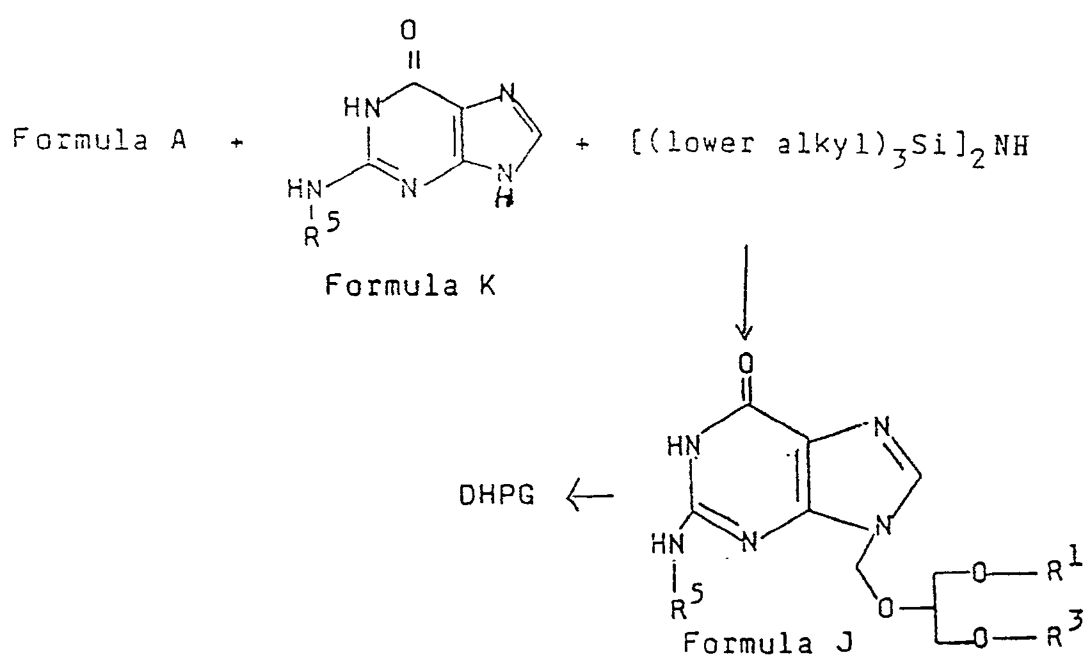


Alternatively, Formula A can be reacted directly with a trialkylsilyl protected guanine to afford the DHPG ester or ether. Guanine or an optionally substituted guanine derivative is heated in an aprotic hydrocarbon

solvent, for example, toluene, or xylenes and the like, with greater than 3 molar equivalents of hexa-lower-alkyl-disilazane, for example, hexamethyldisilazane, hexaethyldisilazane and the like, and a catalyst. The catalyst is selected from the group of trialkyl silyl sulfates, chlorosilanes, ammonium sulfate and pyridine. A volume of volatiles equal to the volume of hydrocarbon solvent is removed by vacuum distillation from the resulting solution. Then compound Formula A is added and the mixture is heated at a temperature greater than ambient temperature until anaylsis of the mixture by TLC shows the consumption of Formula A and the formation of the DHPG ester or ether (Formula J). Treatment of the reaction mixture at this time to remove all the trimethylsilyl groups, for example, by heating with lower alkyl alcohols, for example, methanol, ethanol or iso-propanol, affords the DHPG ester or ether which can be purified by standard methods. If $R^5$ is acyl, then $R^5$ can be removed with, for example, methanolic ammonia to obtain the DHPG ester or ether. If DHPG is desired, Formula J can be deprotected ($R^1$, $R^3$, and $R^5$) by reaction with a mixture of, for example, ammonium hydroxide and water.

## EXAMPLES

The following nonlimiting examples show various aspects of this invention.

### EXAMPLE 1

1,3-di(1-adamantanoyl) glycerol

In a flask under nitrogen 1.053 gms of glycerol is dissolved in 4 ml of pyridine and 6 ml of methylene chloride and cooled to about -10° C. Then 5.00 gms of (1-adamantanoyl)-chloride (Aldrich Chemical Co.) is added in one portion and stirring is continued for about half an hour with cooking and then another two hours at ambient temperature. The resulting solid is filtered and washed with methylene chloride. The combined organic layers are washed twice with dilute aqueous HCl and then once with water and dried over magnesium sulfate. 1,3-di(1-adamantanoyl) glycerol is isolated as an oil.

Proton NMR, $CDCl_3$ 1.5-2.2(m, 30H); 2.3-2.7(br. s, 1H); 4.1-4.3(m, 5H).

Using a similar method but substituting the appropriate compound for (1-adamantanoyl)chloride, the following compounds were prepared:

1,3-di-benzoyl glycerol;
1,3-di-pivaloyl glycerol;
1,3-di-butanoyl glycerol;
1,3-di-s-butanoyl glycerol;
1,3-di-t-butanoyl glycerol;
1,3-di-propanoyl glycerol;
1,3-di-hexanoyl glycerol;
1,3-di-heptanoyl glycerol;
1,3-di-octanoyl glycerol;
1,3-di-nonanoyl glycerol;
1,3-di-decanoyl glycerol;
1,3-di-undecanoyl glycerol;
1,3-di-dodecanoyl glycerol;
1,3-di-tridecanoyl glycerol;
1,3-di-tetradecanoyl glycerol;
1,3-di-pentadecanoyl glycerol;
1,3-di-hexadecanoyl glycerol;
1,3-di-heptadecanoyl glycerol;
1,3-di-octadecanoyl glycerol;
1,3-di-nonadecanoyl glycerol;
1,3-di-eicosanoyl glycerol;
1,3-di-p-chlorobenzoyl glycerol;
1,3-di-o-chlorobenzoyl glycerol;
1,3-di-m-chlorobenzoyl glycerol;
1,3-di-p-bromobenzoyl glycerol;
1,3-di-o-bromobenzoyl glycerol;
1,3-di-m-bromobenzoyl glycerol;
1,3-di-p-fluorobenzoyl glycerol;

1,3-di-o-fluorobenzoyl glycerol;
1,3-di-m-fluorobenzoyl glycerol;
1,3-di-p-iodobenzoyl glycerol;
1,3-di-o-iodobenzoyl glycerol;
1,3-di-m-iodobenzoyl glycerol;
1,3-di-toluoyl glycerol;
1,3-di-phenylacetyl glycerol;
1,3-di-3-phenylpropanoyl glycerol;
1,3-di-4-phenylbutanoyl glycerol.

The 1,3-di-alkyl glycerols can be prepared according to the method described in US-A-4 355 032.

EXAMPLE 2

1,3-di-isopropyl-2-propanoyloxymethyl glycerol

154 g of 1,3-di-isopropyl glycerol and 616 ml of methoxymethylpropionate plus 9.98 g para-toluene sulfonic acid were mixed in a 2-liter round bottom flask equipped with magnetic stirring and an internal thermometer. A 6° C temperature rise occurred over 10 minutes. This was followed by a steady drop in temperature back to ambient temperature. A TLC (25% ethyl acetate / 75% hexane rf=0.69, silica) check after 45 minutes of reaction showed a nearly complete consumption of the starting alcohol.

The reaction mixture was added to a separatory funnel with 500 ml of hexane as an organic phase followed by washing the organic layer with 500 ml of water and washing it twice with 500 ml of saturated aqueous sodium bicarbonate and a final 500 ml wash of water. The organic layer was then dried over anhydrous magnesium sulfate and stripped on a rotary evaporator and 1,3-di-isopropyl-2-propanoyloxymethyl glycerol isolated.

Proton NMR, $CDCl_3$, 1.1(complex, 9H); 2.3(g; 2H); 3.4-4.0(complex, 7H); 5.2(s, 2H).

Similarly, substituting the appropriate compound of Example 1, the following compounds were prepared:
1,3-di-methyl-2-propanoyloxymethyl glycerol;
1,3-di-ethyl-2-propanoyloxymethyl glycerol;
1,3-di-propyl-2-propanoyloxymethyl glycerol;
1,3-di-butyl-2-propanoyloxymethyl glycerol;
1,3-di-s-butyl-2-propanoyloxymethyl glycerol;
1,3-di-t-butyl-2-propanoyloxymethyl glycerol;
1,3-di-phenyl-2-propanoyloxymethyl glycerol;
1,3-di-benzyl-2-propanoyloxymethyl glycerol.

In a similar manner, methoxymethylbutanoate, or methoxymethylpentanoate can be used to prepare the appropriate 1,3-di-alkyl-2-alkanoyloxymethyl glycerol.

EXAMPLE 3

1,3-dipropanoyl-2-propanoyloxymethyl glycerol

339 g of propionic anhydride and 185 g of the crude oil produced from the reaction of Example 2 (1,3-di-isopropyl-2-propanoyloxymethyl glycerol) were added to a 2-liter, 3-neck, round bottom flask equipped with a magnetic stirrer, a reflux condenser, an internal thermometer and a nitrogen inlet set up so that heating or cooling could be applied rapidly. 17.9 g of borontrifluoride etherate was then added in one portion. The reaction temperature immediately climbed from about 20° C (ambient) to about 50° C at which point cooling was applied. The reaction temperature was carefully kept between 50° C and 60° C by heating or cooling as necessary. Reaction progress was followed by TLC (25% ethyl acetate / 75% hexane rf=0.31, silica)of the reaction mixture.

After 100 minutes the reaction mixture was added to a separatory funnel with 500 ml of toluene. The organic layer was washed with 500 ml of saturated aqueous sodium carbonate and twice with 500 ml of water. The organic layer was then dried over anhydrous sodium sulfate filtered and stripped on a rotary evaporator under a 88hPa(26 inches of mercury)vacuum. A crude black oil was recovered and then purified by 2 passes through a wiped film distillation apparatus. The purified oil was 1,3-dipropanoyl-2-propanoyloxymethyl glycerol.

Proton NMR, $CDCl_3$, 1.1(t, 9H); 2.3(g, 6H); 4.1(complex, 5H); 5.2(s, 2H).

In a similar manner the appropriate 1,3-di-alkyl-2-propanoyloxymethyl glycerols of Example 2 can be

reacted to prepare 1,3-di-propanoyl-2-propanoyloxymethyl glycerol.

In a similar manner the appropriate 1,3-di-alkyl-2-acetyloxymethyl glycerol, 1,3-di-alkyl-2-butanoyloxymethyl glycerol, or 1,3-di-alkyl-2-pentanoyloxymethyl glycerol can be reacted to form the corresponding 1,3-di-acyl compound.

EXAMPLE 4

1,3-dibenzyl-2-acetoxymethyl glycerol

75 9 di-benzyl glycerol, 5.25 g para-toluene sulfonic acid, 300 ml hexane, and 300 ml methoxymethyl acetate were added to a 2-1 round bottom flask with magnetic stirring, internal thermometer and drying tube. The reaction mixture was brought to 60-65° C for 90 minutes, followed by TLC (20% ethyl acetate/ 80% hexane rf=0.31, silica)followed by cooling, transferring to a separatory funnel, washing once with 700 ml $H_2O$, and once with 700 ml saturated aqueous sodium carbonate solution. 150 ml $CH_2Cl_2$ was then added followed by a final wash with 700 ml $H_2O$. The organic layer was dried over sodium sulfate, filtered, and stripped at high vacuum to give 1,3-dibenzyl-2-acetoxymethyl glycerol as a clear oil.

Proton NMR, $CDCl_3$ 1.9(s, 3H); 3.6(complex, 4H); 4.0(m, 1H); 4.5(s, 4H); 5.2(s, 2H); 7.2(complex, 10H)

Similarly, using the appropriate 1,3-di-substituted glycerol from Example 1, the following compounds were prepared:

1,3-di-methyl-2-acetoxymethyl glycerol;
1,3-di-ethyl-2-acetoxymethyl glycerol;
1,3-di-propyl-2-acetoxymethyl glycerol;
1,3-di-isopropyl-2-acetoxymethyl glycerol;
1,3-di-butyl-2-acetoxymethyl glycerol;
1,3-di-s-butyl-2-acetoxymethyl glycerol;
1,3-di-t-butyl-2-acetoxymethyl glycerol;
1,3-di-phenyl-2-acetoxymethyl glycerol.

EXAMPLE 5

1,3-dipivaloyl-2-methoxymethyl glycerol

50.45 g of 1,3-dipivaloyl glycerol was dissolved in 200 ml of methoxymethyl acetate. After the addition of 2.5 g of p-toluenesulfonic acid monohydrate, the mixture was stirred for 1 hr then diluted with 200 ml of hexane and washed twice with 200 ml saturated aqueous sodium carbonate, once with aqueous NaCl solution, dried over anhydrous magnesium sulfate and concentrated in vacuo to afford 1,3-dipivaloyl-2-methoxymethyl glycerol as a colorless oil.

Proton NMR, $CDCl_3$, 1.2(s, 18H); 3.4(s, 3H); 4.1(m, 1H); 4.2(m, 4H); 4.7(s, 2H).

Similarly using the appropriate 1,3 di-substituted glycerols from Example 1, the following compounds were prepared:

1,3-di(1-adamantanoyl)-2-methoxymethyl glycerol

Proton NMR, $CDCl_3$, 1.6-2.1(complex, 30H); 3.4(s, 3H); 4.1(complex, 1H); 4.2(complex, 4H); 4.8(s, 2H).

1,3-di-benzoyl-2-methoxymethyl glycerol;
1,3-di-pivaloyl-2-methoxymethyl glycerol;
1,3-di-butanoyl-2-methoxymethyl glycerol;
1,3-di-s-butanoyl-2-methoxymethyl glycerol;
1,3-di-t-butanoyl-2-methoxymethyl glycerol;
1,3-di-propanoyl-2-methoxymethyl glycerol;
1,3-di-hexanoyl-2-methoxymethyl glycerol;
1,3-di-heptanoyl-2-methoxymethyl glycerol;
1,3-di-octanoyl-2-methoxymethyl glycerol;
1,3-di-nonanoyl-2-methoxymethyl glycerol;
1,3-di-decanoyl-2-methoxymethyl glycerol;
1,3-di-undecanoyl-2-methoxymethyl glycerol;
1,3-di-dodecanoyl-2-methoxymethyl glycerol;
1,3-di-tridecanoyl-2-methoxymethyl glycerol;
1,3-di-tetradecanoyl-2-methoxymethyl glycerol;
1,3-di-pentadecanoyl-2-methoxymethyl glycerol;

1,3-di-hexadecanoyl-2-methoxymethyl glycerol;
1,3-di-heptadecanoyl-2-methoxymethyl glycerol;
1,3-di-octadecanoyl-2-methoxymethyl glycerol;
1,3-di-nonadecanoyl-2-methoxymethyl glycerol;
1,3-di-eicosanoyl-2-methoxymethyl glycerol;
1,3-di-p-chlorobenzoyl-2-methoxymethyl glycerol:
1,3-di-o-chlorobenzoyl-2-methoxymethyl glycerol;
1,3-di-m-chlorobenzoyl-2-methoxymethyl glycerol;
1,3-di-p-bromobenzoyl-2-methoxymethyl glycerol;
1,3-di-o-bromobenzoyl-2-methoxymethyl glycerol;
1,3-di-m-bromobenzoyl-2-methoxymethyl glycerol;
1,3-di-p-fluorobenzoyl-2-methoxymethyl glycerol;
1,3-di-o-fluorobenzoyl-2-methoxymethyl glycerol;
1,3-di-m-fluorobenzoyl-2-methoxymethyl glycerol;
1,3-di-p-iodobenzoyl-2-methoxymethyl glycerol;
1,3-di-o-iodobenzoyl-2-methoxymethyl glycerol;
1,3-di-m-iodobenzoyl-2-methoxymethyl glycerol;
1,3-di-toluoyl-2-methoxymethyl glycerol;
1,3-di-phenylacetyl-2-methoxymethyl glycerol;
1,3-di-3-phenylpropanoyl-2-methoxymethyl glycerol;
1,3-di-4-phenylbutanoyl-2-methoxymethyl glycerol.

In a similar manner ethoxymethylacetate, propoxymethylacetate, or butoxymethylacetate can be substituted to prepare the corresponding 1,3-di-acyl-2-alkoxymethyl glycerol.

## EXAMPLE 6

### 1,3-dipivaloyl-2-acetoxymethyl glycerol

50 g of 1,3-dipivaloyl-2-methoxymethyl glycerol, 150 ml of methylene chloride, and 17 ml of acetic anhydride was cooled to about -5° C. Then 0.3 ml of boron triflouride etherate was added and the mixture was stirred for an additional 1 hr at about 0° C then allowed to warm to 25° C over an additional 1.5 hr. After this period, 100 ml saturated aqueous $Na_2CO_3$ was added to the mixture followed by 100 ml $CH_2Cl_2$. After vigorous stirring for 5 min, the organic layer was separated and washed once with 100 ml $H_2O$, once with aqueous NaCl, dried over anhydrous magnesium sulfate and concentrated *in vacuo* to afford 1,3-dipivaloyl-2-acetoxymethyl glycerol as a faintly colored oil.

Proton NMR, $CDCl_3$, 1.2(s, 18H); 2.1(s, 3H); 4.1-4.3(m, 5H); 5.3(s, 2H); 4.7(s, 2H).

Similarly 1,3-di(1-adamantanoyl)-2-acetoxymethyl glycerol, was made.

Proton NMR, $CDCl_3$, 1.6-2.1(complex, 30H); 2.1(s, 3H); 4.1(complex, 5H); 5.3(s, 2H).

1,3-di-benzoyl-2-acetoxymethyl glycerol;
1,3-di-pivaloyl-2-acetoxymethyl glycerol;
1,3-di-butanoyl-2-acetoxymethyl glycerol;
1,3-di-s-butanoyl-2-acetoxymethyl glycerol;
1,3-di-t-butanoyl-2-acetoxymethyl glycerol;
1,3-di-propanoyl-2-acetoxymethyl glycerol;
1,3-di-hexanoyl-2-acetoxymethyl glycerol;
1,3-di-heptanoyl-2-acetoxymethyl glycerol;
1,3-di-octanoyl-2-acetoxymethyl glycerol;
1,3-di-nonanoyl-2-acetoxymethyl glycerol;
1,3-di-decanoyl-2-acetoxymethyl glycerol;
1,3-di-undecanoyl-2-acetoxymethyl glycerol;
1,3-di-dodecanoyl-2-acetoxymethyl glycerol;
1,3-di-tridecanoyl-2-acetoxymethyl glycerol;
1,3-di-tetradecanoyl-2-acetoxymethyl glycerol;
1,3-di-pentadecanoyl-2-acetoxymethyl glycerol;
1,3-di-hexadecanoyl-2-acetoxymethyl glycerol;
1,3-di-heptadecanoyl-2-acetoxymethyl glycerol;
1,3-di-octadecanoyl-2-acetoxymethyl glycerol;
1,3-di-nonadecanoyl-2-acetoxymethyl glycerol;

1,3-di-eicosanoyl-2-acetoxymethyl glycerol;
1,3-di-p-chlorobenzoyl-2-acetoxymethyl glycerol;
1,3-di-o-chlorobenzoyl-2-acetoxymethyl glycerol;
1,3-di-m-chlorobenzoyl-2-acetoxymethyl glycerol;
1,3-di-p-bromobenzoyl-2-acetoxymethyl glycerol;
1,3-di-o-bromobenzoyl-2-acetoxymethyl glycerol:
1,3-di-m-bromobenzoyl-2-acetoxymethyl glycerol;
1,3-di-p-fluorobenzoyl-2-acetoxymethyl glycerol;
1,3-di-o-fluorobenzoyl-2-acetoxymethyl glycerol;
1,3-di-m-fluorobenzoyl-2-acetoxymethyl glycerol;
1,3-di-p-iodobenzoyl-2-acetoxymethyl glycerol;
1,3-di-o-iodobenzoyl-2-acetoxymethyl glycerol;
1,3-di-m-iodobenzoyl-2-acetoxymethyl glycerol;
1,3-di-toluoyl-2-acetoxymethyl glycerol;
1,3-di-phenylacetyl-2-acetoxymethyl glycerol;
1,3-di-3-phenylpropanoyl-2-acetoxymethyl glycerol;
1,3-di-4-phenylbutanoyl-2-acetoxymethyl glycerol.

In a similar manner ethoxyethylacetate, propoxymethylacetate, or butoxymethylacetate can be substituted to prepare the corresponding 1,3-di-acyl-2-acyloxymethyl glycerol.

## EXAMPLE 7

## ETHERS AND ESTERS OF DHPG

### Dipivaloyl DHPG

8.00 g of guanine, 40 ml hexamethyldisilazane, 80 ml of xylene and 0.64 g of ammonium sulfate are refluxed for about 20 hours. 80 ml of volatiles are then distilled out of the resulting clear solution and discarded. 23.1 g of 1,3-dipivaloyl-2-acetoxymethyl glycerol is then added to the remaining solution and the mixture is refluxed for about 20 hours. After cooling the volatiles are removed in vacuo. The residue is refluxed with 40 ml of isopropanol and then stripped of volatiles in vacuo and then chromatographed over silica gel yielding dipivaloyl DHPG.

Proton NMR, DMS0-$d_6$ 1.1(s, 18H); 3.9-4.3(m, 5H); 5.4(s, 2H); 6.4(s, 2H); 7.8(s, 1H); 10.68(s, 1H).

Similarly 1,3-dibenzyl DHPG

Proton NMR, DMSO-$d_6$ 3.5(m, 4H); 4.1(m, 1H); 4.4(s, 4H); 5.5(s, 2H); 6.7(s, 2H); 7.3(complex, 10H); 7.9-(s, 1H); 10.9(s, 1H) was made using 1,3-dibenzyl-2-acetoxymethyl-glycerol as the starting material.

In a similar manner using the appropriate 1,3-dialkyl- or 1,3-diacyl-2-alkanoyloxymethyl glycerol the following ethers and esters of DHPG can be prepared:
9-(1,3-dimethyloxy-2-propoxymethyl)-guanine;
9-(1,3-diethyloxy-2-propoxymethyl)-guanine;
9-(1,3-dipropyloxy-2-propoxymethyl)-guanine;
9-(1,3-diisopropyloxy-2-propoxymethyl)-guanine;
9-(1,3-dibutyloxy-2-propoxymethyl)-guanine;
9-(1,3-di-s-butyloxy-2-propoxymethyl)-guanine;
9-(1,3-di-t-butyloxy-2-propoxymethyl)-guanine;
9-(1,3-diphenyloxy-2-propoxymethyl)-guanine;
9-(1,3-dibenzoyl-2-propoxymethyl)-guanine;
9-(1,3-dibutanoyl-2-propoxymethyl)-guanine;
9-(1,3-di-s-butanoyl-2-propoxymethyl)-guanine;
9-(1,3-di-t-butanoyl-2-propoxymethyl)-guanine;
9-(1,3-di-propanoyl-2-propoxymethyl)-guanine;
9-(1,3-di-hexanoyl-2-propoxymethyl)-guanine;
9-(1,3-di-heptanoyl-2-propoxymethyl)-guanine;
9-(1,3-di-octanoyl-2-propoxymethyl)-guanine;
9-(1,3-di-nonanoyl-2-propoxymethyl)-guanine;
9-(1,3-di-decanoyl-2-propoxymethyl)-guanine;
9-(1,3-di-undecanoyl-2-propoxymethyl)-guanine;
9-(1,3-di-dodecanoyl-2-propoxymethyl)-guanine;

9-(1,3-di-tridecanoyl-2-propoxymethyl)-guanine;
9-(1,3-di-tetradecanoyl-2-propoxymethyl)-guanine;
9-(1,3-di-pentadecanoyl-2-propoxymethyl)-guanine;
9-(1,3-di-hexadecanoyl-2-propoxymethyl)-guanine;
9-(1,3-di-heptadecanoyl-2-propoxymethyl)-guanine;
9-(1,3-di-octadecanoyl-2-propoxymethyl)-guanine;
9-(1,3-di-nonadecanoyl-2-propoxymethyl)-guanine;
9-(1,3-di-eicosanoyl-2-propoxymethyl)-guanine;
9-(1,3-di-p-chlorobenzoyl-2-propoxymethyl)-guanine;
9-(1,3-di-o-chlorobenzoyl-2-propoxymethyl)-guanine;
9-(1,3-di-m-chlorobenzoyl-2-propoxymethyl)-guanine;
9-(1,3-di-p-bromobenzoyl-2-propoxymethyl)-guanine;
9-(1,3-di-o-bromobenzoyl-2-propoxymethyl)-guanine;
9-(1,3-di-m-bromobenzoyl-2-propoxymethyl)-guanine;
9-(1,3-di-p-fluorobenzoyl-2-propoxymethyl)-guanine;
9-(1,3-di-o-fluorobenzoyl-2-propoxymethyl)-guanine;
9-(1,3-di-m-fluorobenzoyl-2-propoxymethyl)-guanine;
9-(1,3-di-p-iodobenzoyl-2-propoxymethyl)-guanine;
9-(1,3-di-o-iodobenzoyl-2-propoxymethyl)-guanine;
9-(1,3-di-m-iodobenzoyl-2-propoxymethyl)-guanine;
9-(1,3-di-toluoyl-2-propoxymethyl)-guanine;
9-(1,3-di-phenylacetyl-2-propoxymethyl)-guanine;
9-(1,3-di-phenylpropanoyl-2-propoxymethyl)-guanine;
9-(1,3-di-phenylbutanoyl-2-propoxymethyl)-guanine.

## Claims

**Claims for the following contracting states: BE, FR, CH, DE, GB, IT, LI, LU, NL, SE**

**1.** A process for preparing a compound represented by formula A

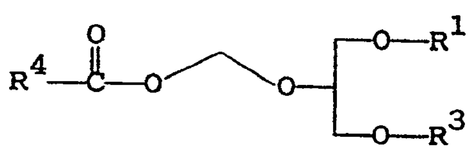

A

wherein $R^1$ and $R^3$ are selected from hydrogen, optionally substituted lower alkyl, acyl, 1-adamantanoyl, optionally substituted phenyl or optionally substituted phenyl lower alkyl and $R^4$ is optionally substituted lower alkyl, comprising:

a) reacting a compound of formula D

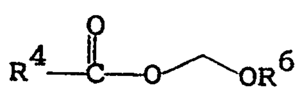

D

wherein $R^4$ is as defined above and $R^6$ is optionally substituted lower alkyl, with a compound of formula E

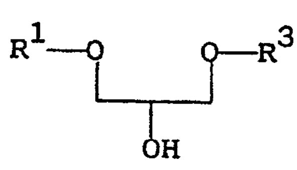

E

wherein $R^1$ and $R^3$ are as defined above, in the presence of a protic acid without external heat, to

form a compound of formula C

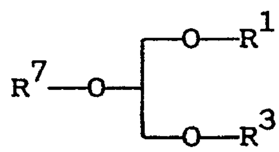

C

wherein $R^1$ and $R^3$ are as defined above and $R^7$ is $R^4C(O)OCH_2$ or $R^6OCH_2$ wherein $R^4$ and $R^6$ are as defined above;

b) optionally followed by, if $R^7$ is $R^6OCH_2$, reacting a compound represented by formula B

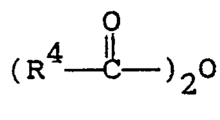

B

wherein $R^4$ is independently selected from the group as defined above, with a compound of formula C in which $R^7$ is $R^6OCH_2$, in the presence of a Lewis acid for about 60 to 180 minutes at a temperature of between 45° and 65° C.

**2.** The process of claim 1 wherein, in the compound represented by formula E, $R^1$ and $R^3$ are both benzyl.

**3.** The process of claim 1 wherein, in the compound represented by formula B, $R^4$ is methyl.

**4.** The process of claim 1, wherein the compound represented by formula E is a compound wherein $R^1$ and $R^3$ are both (1-adamantanoyl)- and the compound represented by formula B is acetic anhydride.

**5.** The process of claim 1 wherein the compound represented by formula E is a compound wherein $R^1$ and $R^3$ are both pivaloyl and the compound represented by formula B is acetic anhydride.

**6.** A compound represented by the formula

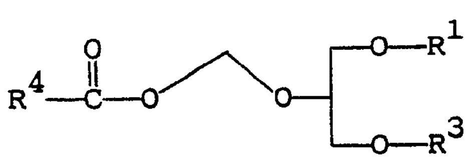

Formula A

wherein $R^1$ and $R^3$ are selected from hydrogen, optionally substituted lower alkyl, acyl, 1-adamantanoyl, optionally substituted phenyl or optionally substituted phenyl lower alkyl and $R^4$ is optionally substituted lower alkyl; except when $R^4$ is methyl, at least one of $R^1$ and $R^3$ is not acetyl, lower alkyl, optionally substituted phenyl or optionally substituted phenyl lower alkyl; and when $R^4$ is ethyl, $R^1$ and $R^3$ cannot both be the same lower alkyl group.

**7.** A compound of claim 6 wherein $R^1$ and $R^3$ are the same.

**8.** A compound of claim 7 wherein $R^4$ is methyl.

**9.** A compound of claim 8 which is 1,3-dipivaloyl-2-acetoxymethoxy glycerol or 1,3-di-(1-adamantanoyl)-2-acetoxymethoxy glycerol.

**10.** A compound of claim 7, wherein $R^4$ is ethyl.

**11.** A compound of claim 10 which is 1,3-dipivaloyl-2-propanoyloxymethyl glycerol or 1,3-di-(1-adamantanoyl)-2-propanoyloxymethyl glycerol.

**12.** A compound represented by the formula

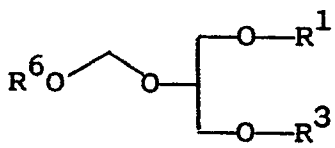

**Formula F**

wherein $R^1$, $R^3$ and $R^6$ are defined as optionally substituted lower alkyl, except that $R^1$ and $R^3$ cannot both be propyl.

**13.** A method for making compounds represented by the formula

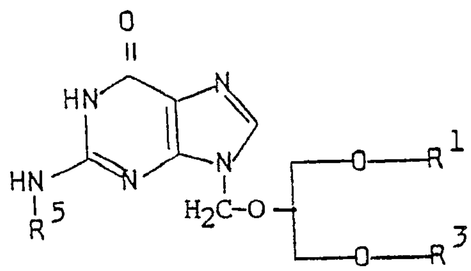

wherein $R^1$ and $R^3$ are selected from hydrogen, optionally substituted lower alkyl, acyl, 1-adamantanoyl, optionally substitued phenyl or optionally substituted phenyl lower alkyl and $R^5$ is optionally substituted acyl or hydrogen, comprising:

a) reacting optionally substituted guanine with excess hexa-lower alkyl disilazane and a catalyst, and with a compound represented by the formula:

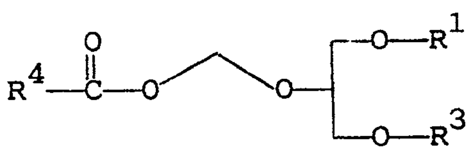

**Formula A**

wherein $R^1$ and $R^3$ are selected from hydrogen, optionally substituted lower alkyl, 1-adamantanoyl, acyl, optionally substituted phenyl or optionally substituted phenyl lower alkyl and $R^4$ is defined as optionally substituted lower alkyl; followed by

b) treating the resulting mixture with a lower alkyl alcohol; optionally followed by

c) if $R^1$ and $R^3$ are not hydrogen, treating the resulting mixture with ammonium hydroxide and water.

**14.** A method for making compounds represented by the formula

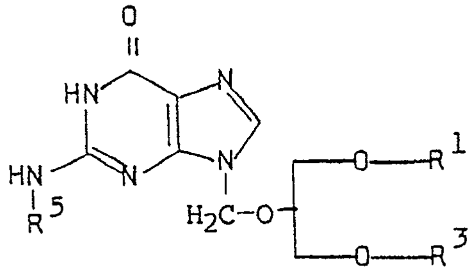

wherein $R^1$ and $R^3$ are hydrogen, acyl or 1-adamantanoyl and $R^5$ is optionally substituted acyl or hydrogen, comprising:

a) reacting optionally substituted guanine in the presence of an acidic catalyst with a compound represented by the formula:

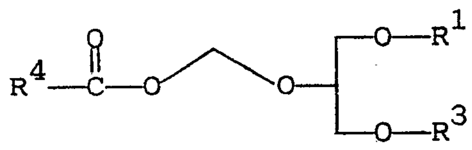

**Formula A**

wherein $R^1$ and $R^3$ are selected from acyl or 1-adamantanoyl and $R^4$ is defined as optionally substituted lower alkyl; optionally followed by

b) treating the resulting mixture with a lower alkyl alcohol; and/or

c) if $R^1$ and $R^3$ are not hydrogen, treating the resulting mixture with ammonium hydroxide and water.

**15.** A method for making compounds represented by the formula

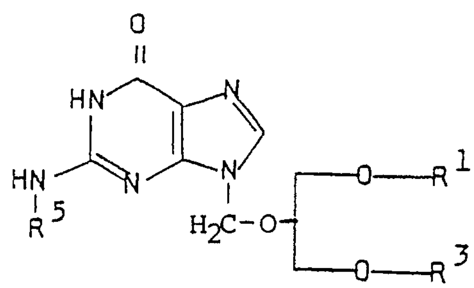

wherein $R^1$ and $R^3$ are selected from hydrogen, optionally substituted lower alkyl, acyl, 1-adamantanoyl, optionally substituted phenyl or optionally substituted phenyl lower alkyl and $R^5$ is optionally substituted acyl or hydrogen, comprising:

a) the preparation according to claim 1 of a compound of the formula

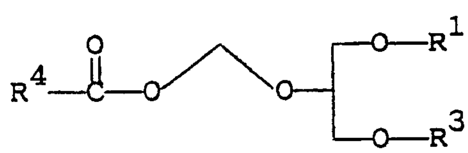

**Formula A**

wherein $R^1$ and $R^3$ are selected from hydrogen, optionally substituted lower alkyl, 1-adamantanoyl, acyl, optionally substituted phenyl or optionally substituted phenyl lower alkyl and $R^4$ is defined as optionally substituted lower alkyl; followed by

b) reacting the thus prepared compound of formula A with optionally substituted guanine; optionally followed by

c) treating the resulting mixture with a lower alkyl alcohol; and/or

d) if $R^1$ and $R^3$ are not hydrogen, treating the resulting mixture with ammonium hydroxide and water.

**16.** The method of any one of claims 13 to 15, wherein unsubstituted guanine is used.

**17.** The method of any one of claims 13 to 16 wherein $R^5$ is acyl.

**18.** The method of any one of claims 13 to 17 wherein $R^1$ and $R^3$ are the same.

**19.** The method of claim 18 wherein $R^1$ and $R^3$ are 1-adamantanoyl or pivaloyl.

**Claims for the following contracting state: AT**

**1.** A process for preparing a compound represented by formula A

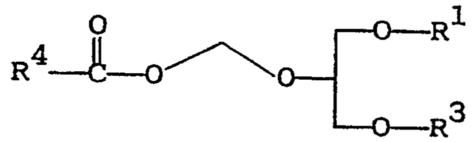

A

wherein $R^1$ and $R^3$ are selected from hydrogen, optionally substituted lower alkyl, acyl, 1-adamantanoyl, optionally substituted phenyl or optionally substituted phenyl lower alkyl and $R^4$ is optionally substituted lower alkyl, comprising:

a) reacting a compound of formula D

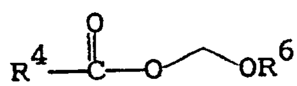

D

wherein $R^4$ is as defined above and $R^6$ is optionally substituted lower alkyl, with a compound of formula E

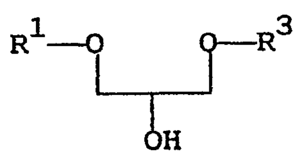

E

wherein $R^1$ and $R^3$ are as defined above, in the presence of a protic acid without external heat, to form a compound of formula C

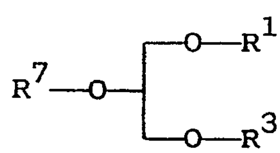

C

wherein $R^1$ and $R^3$ are as defined above and $R^7$ is $R^4C(O)OCH_2$ or $R^6OCH_2$ wherein $R^4$ and $R^6$ are as defined above;

b) optionally followed by, if $R^7$ is $R^6OCH_2$, reacting a compound represented by formula B

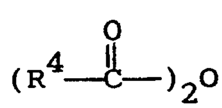

B

wherein $R^4$ is independently selected from the group as defined above, with a compound of formula C in which $R^7$ is $R^6OCH_2$, in the presence of a Lewis acid for about 60 to 180 minutes at a temperature of between 45° and 65° C.

2. The process of claim 1 wherein, in the compound represented by formula E, $R^1$ and $R^3$ are both benzyl.

3. The process of claim 1 wherein, in the compound represented by formula B, $R^4$ is methyl.

4. The process of claim 1, wherein the compound represented by formula E is a compound wherein $R^1$ and $R^3$ are both (1-adamantanoyl)- and the compound represented by formula B is acetic anhydride.

5. The process of claim 1 wherein the compound represented by formula E is a compound wherein $R^1$ and $R^3$ are both pivaloyl and the compound represented by formula B is acetic anhydride.

6. The process of claim 1 wherein a compound of formula A is prepared in which $R^1$ and $R^3$ are selected from hydrogen, optionally substituted lower alkyl, acyl, 1-adamantanoyl, optionally substituted phenyl or optionally substituted phenyl lower alkyl and $R^4$ is optionally substituted lower alkyl; except when $R^4$ is methyl, at least one of $R^1$ and $R^3$ is not acetyl, lower alkyl, optionally substituted phenyl or optionally substituted phenyl lower alkyl; and when $R^4$ is ethyl, $R^1$ and $R^3$ cannot both be the same lower alkyl group.

7. The process of claim 6 wherein a compound is prepared in which $R^1$ and $R^3$ are the same.

8. The process of claim 7 wherein a compound is prepared in which $R^4$ is methyl.

9. The process of claim 8 wherein 1,3-dipivaloyl-2-acetoxymethoxy glycerol or 1,3-di-(1-adamantanoyl)-2-acetoxymethoxy glycerol is prepared.

10. The process of claim 7 wherein a compound is prepared in which $R^4$ is ethyl.

11. The process of claim 10 wherein 1,3-dipivaloyl-2-propanoyloxymethyl glycerol or 1,3-di-(1-adamantanoyl)-2-propanoyloxymethyl glycerol is prepared.

12. The process of claim 1 wherein a compound of formula F

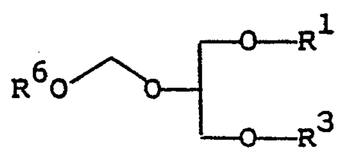

Formula F

is prepared in which $R^1$, $R^3$ and $R^6$ are defined as optionally substituted lower alkyl, except that $R^1$ and $R^3$ cannot both be propyl.

13. A method for making compounds represented by the formula

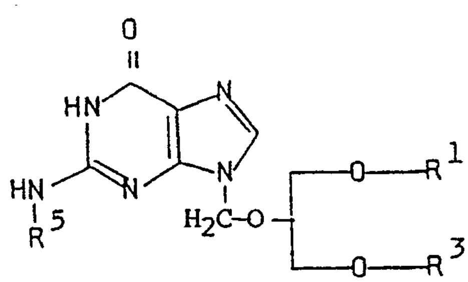

wherein $R^1$ and $R^3$ are selected from hydrogen, optionally substituted lower alkyl, acyl, 1-adamantanoyl, optionally substitued phenyl or optionally substituted phenyl lower alkyl and $R^5$ is optionally substituted acyl or hydrogen, comprising:

a) reacting optionally substituted guanine with excess hexa-lower alkyl disilazane and a catalyst, and with a compound represented by the formula:

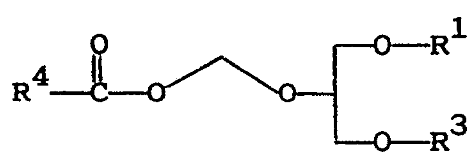

Formula A

wherein $R^1$ and $R^3$ are selected from hydrogen, optionally substituted lower alkyl, 1-adamantanoyl, acyl, optionally substituted phenyl or optionally substituted phenyl lower alkyl and $R^4$ is defined as optionally substituted lower alkyl; followed by

b) treating the resulting mixture with a lower alkyl alcohol; optionally followed by

c) if $R^1$ and $R^3$ are not hydrogen, treating the resulting mixture with ammonium hydroxide and water.

**14.** A method for making compounds represented by the formula

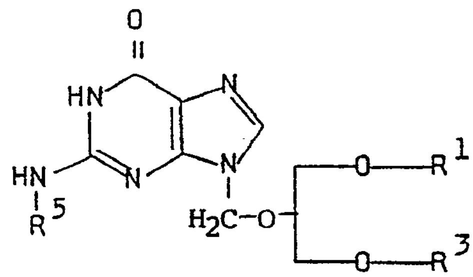

wherein $R^1$ and $R^3$ are hydrogen, acyl or 1-adamantanoyl and $R^5$ is optionally substituted acyl or hydrogen, comprising:

a) reacting optionally substituted guanine in the presence of an acidic catalyst with a compound represented by the formula:

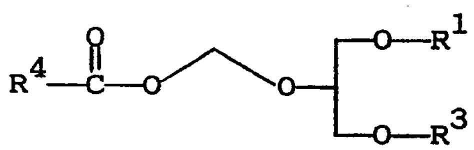

**Formula A**

wherein $R^1$ and $R^3$ are selected from acyl or 1-adamantanoyl and $R^4$ is defined as optionally substituted lower alkyl; optionally followed by

b) treating the resulting mixture with a lower alkyl alcohol; and/or

c) if $R^1$ and $R^3$ are not hydrogen, treating the resulting mixture with ammonium hydroxide and water.

**15.** A method for making compounds represented by the formula

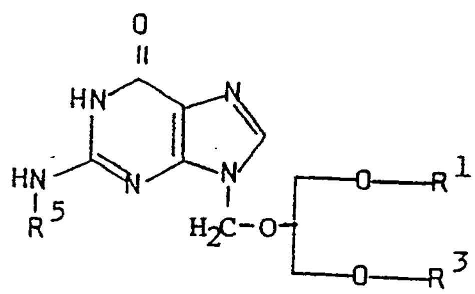

wherein $R^1$ and $R^3$ are selected from hydrogen, optionally substituted lower alkyl, acyl, 1-adamantanoyl, optionally substituted phenyl or optionally substituted phenyl lower alkyl and $R^5$ is optionally substituted acyl or hydrogen, comprising:

a) the preparation according to claim 1 of a compound of the formula

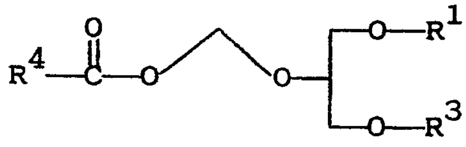

**Formula A**

wherein $R^1$ and $R^3$ are selected from hydrogen, optionally substituted lower alkyl, 1-adamantanoyl, acyl, optionally substituted phenyl or optionally substituted phenyl lower alkyl and $R^4$ is defined as optionally substituted lower alkyl; followed by

b) reacting the thus prepared compound of formula A with optionally substituted guanine; optionally followed by

c) treating the resulting mixture with a lower alkyl alcohol; and/or

d) if $R^1$ and $R^3$ are not hydrogen, treating the resulting mixture with ammonium hydroxide and water.

**16.** The method of any one of claims 13 to 15, wherein unsubstituted guanine is used.

**17.** The method of any one of claims 13 to 16 wherein $R^5$ is acyl.

**18.** The method of any one of claims 13 to 17 wherein $R^1$ and $R^3$ are the same.

**19.** The method of claim 18 wherein $R^1$ and $R^3$ are 1-adamantanoyl or pivaloyl.

**Revendications**

**Revendications pour les Etats contractants suivants: BE, FR, CH, DE, GB, IT, LI, LU, NL, SE**

**1.** Procédé de préparation d'un composé représenté par la formule A

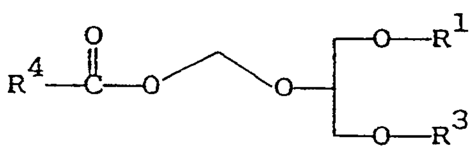

A

dans laquelle $R^1$ et $R^3$ sont choisis entre l'hydrogène, un groupe alkyle inférieur facultativement substitué, acyle, 1-adamantanoyle, phényle facultativement substitué ou (phényle facultativement substitué)-alkyle inférieur, et $R^4$ est un groupe alkyle inférieur facultativement substitué, comprenant :

a) la réaction d'un composé de formule D

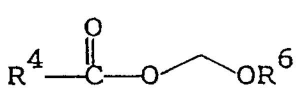

D

dans laquelle $R^4$ est tel que défini ci-dessus et R6 est un groupe alkyle inférieur facultativement substitué, avec un composé de formule E

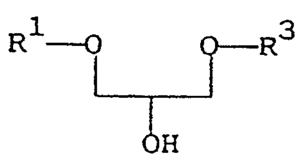

E

dans laquelle $R^1$ et $R^3$ sont tels que définis ci-dessus, en présence d'un acide protique sans apport de chaleur externe, pour former un composé de formule C

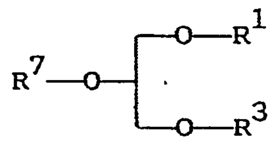

C

dans laquelle $R^1$ et $R^3$ sont tels que définis ci-dessus et $R^7$ est un groupe $R^4C(O)OCH_2$ ou $R^6OCH_2$ dans lequel $R^4$ et $R^6$ sont tels que définis ci-dessus ;

b) facultativement suivie, si $R^7$ est un groupe $R^6OCH_2$, de la réaction d'un composé représenté par la formule B

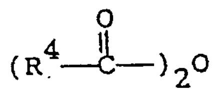

B

dans laquelle $R^4$ est choisi indépendamment dans le groupe tel que défini ci-dessus, avec un composé de formule C dans lequel $R^7$ est un groupe $R^6OCH_2$, en présence d'un acide de Lewis pendant environ 60 à 180 minutes à une température comprise entre 45 et 65 °C.

**2.** Procédé suivant la revendication 1, dans lequel $R^1$ et $R^3$ sont tous deux des groupes benzyle dans le composé représenté par la formule E.

**3.** Procédé suivant la revendication 1, dans lequel $R^4$ est un groupe méthyle dans le composé représenté par la formule B.

**4.** Procédé suivant la revendication 1, dans lequel le composé représenté par la formule E est un composé dans lequel $R^1$ et $R^3$ sont tous deux des groupes (1-adamantanoyle) et le composé représenté par la formule B est l'anhydride acétique.

**5.** Procédé suivant la revendication 1, dans lequel le composé représenté par la formule E est un composé dans lequel $R^1$ et $R^3$ sont tous deux des groupes pivaloyle et le composé représenté par la formule B est l'anhydride acétique.

**6.** Composé représenté par la formule

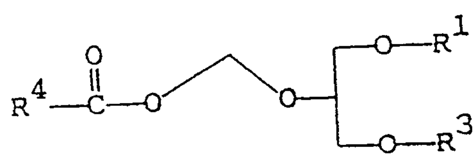

Formule A

dans laquelle $R^1$ et $R^3$ sont choisis entre l'hydrogène, un groupe alkyle inférieur facultativement substitué, acyle, 1-adamantanoyle, phényle facultativement substitué ou (phényle facultativement substitué)-alkyle inférieur, et $R^4$ est un groupe alkyle inférieur facultativement substitué ; excepté que lorsque $R^4$ est un groupe méthyle, l'un au moins de $R^1$ et $R^3$ n'est pas un groupe acétyle, alkyle inférieur, phényle facultativement substitué ou (phényle facultativement substitué)-alkyle inférieur ; et que lorsque $R^4$ est un groupe éthyle, $R^1$ et $R^3$ ne peuvent pas être tous deux le même groupe alkyle inférieur.

**7.** Composé suivant la revendication 6, dans lequel $R^1$ et $R^3$ sont identiques.

**8.** Composé suivant la revendication 7, dans lequel $R^4$ est le groupe méthyle.

**9.** Composé suivant la revendication 8, qui est le 1,3-dipivaloyl-2-acétoxyméthoxyglycérol ou le 1,3-di-(1-adamantanoyl)-2-acétoxyméthoxyglycérol.

**10.** Composé suivant la revendication 7, dans lequel $R^4$ est le groupe éthyle.

**11.** Composé suivant la revendication 10, qui est le 1,3-dipivaloyl-2-propanoyloxyméthylglycérol ou le 1,3-di-(1-adamantanoyl)-2-propanoyloxyméthylglycérol.

**12.** Composé représenté par la formule

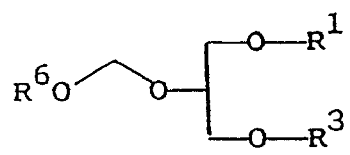

Formule F

dans laquelle $R^1$, $R^3$ et $R^6$ sont définis comme groupes alkyle inférieurs facultativement substitués, excepté que $R^1$ et $R^3$ ne peuvent pas être tous deux des groupes propyle.

**13.** Procédé de préparation de composés représentés par la formule :

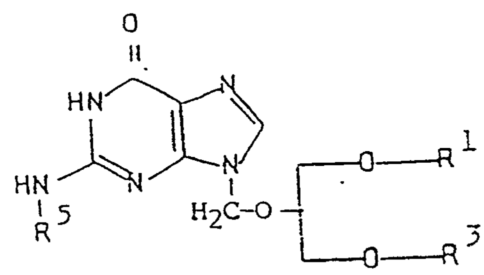

dans laquelle $R^1$ et $R^3$ sont choisis entre l'hydrogène, un groupe alkyle inférieur facultativement substitué, acyle, 1-adamantanoyle, phényle facultativement substitué ou (phényle facultativement substitué)-alkyle inférieur, et $R^5$ est un groupe acyle facultativement substitué ou l'hydrogène, comprenant :

a) la réaction de guanine facultativement substituée avec un excès d'hexa(alkyle inférieur)disilazane et un catalyseur, et avec un composé représenté par la formule :

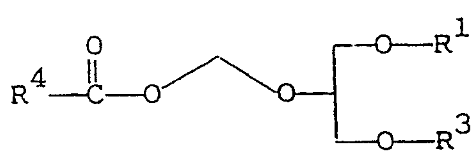

Formule A

dans laquelle $R^1$ et $R^3$ sont choisis entre l'hydrogène, un groupe alkyle inférieur facultativement substitué, 1-adamantanoyle, acyle, phényle facultativement substitué ou (phényle facultativement substitué)-alkyle inférieur, et $R^4$ est défini comme étant un groupe alkyle inférieur facultativement substitué ; suivie

b) du traitement du mélange résultant avec un alcool alkylique inférieur ; éventuellement suivi

c) si $R^1$ et $R^3$ ne sont pas l'hydrogène, d'un traitement du mélange résultant avec l'hydroxyde d'ammonium et l'eau.

**14.** Procédé de préparation de composés représentés par la formule

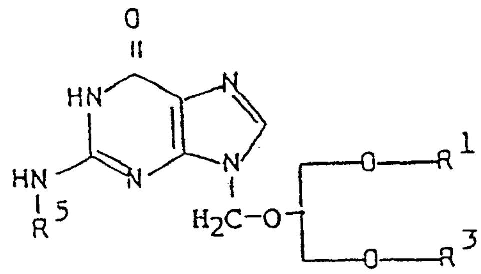

dans laquelle $R^1$ et $R^3$ sont l'hydrogène, des groupes acyle ou 1-adamantanoyle et $R^5$ est un groupe acyle facultativement substitué ou l'hydrogène, comprenant :

a) la réaction de guanine facultativement substituée en présence d'un catalyseur acide avec un composé représenté par la formule

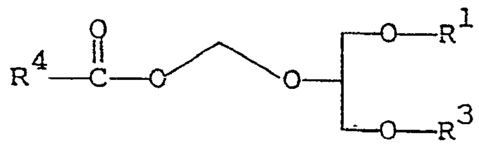

Formule A

dans laquelle $R^1$ et $R^3$ sont choisis entre des groupes acyle ou 1-adamantanoyle, et $R^4$ est défini comme étant un groupe alkyle inférieur facultativement substitué ; éventuellement suivie

b) du traitement du mélange résultant avec un alcool alkylique inférieur ; et/ou

c) si $R^1$ et $R^3$ ne sont pas de l'hydrogène, d'un traitement du mélange résultant avec l'hydroxyde d'ammonium et l'eau.

**15.** Procédé de préparation de composés représentés par la formule

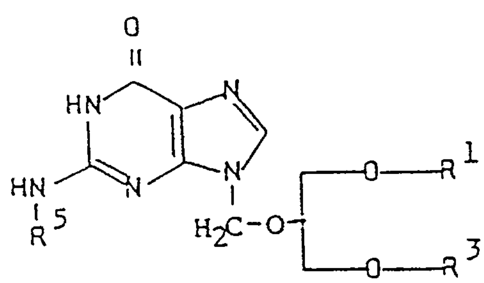

dans laquelle $R^1$ et $R^3$ sont choisis entre l'hydrogène, un groupe alkyle inférieur éventuellement substitué, acyle, 1-adamantanoyle, phényle facultativement substitué ou (phényle facultativement substitué)-alkyle inférieur, et $R^5$ est un groupe acyle facultativement substitué ou l'hydrogène, comprenant :

a) la préparation suivant la revendication 1 d'un composé de formule

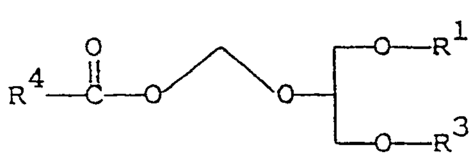

Formule A

dans laquelle $R^1$ et $R^3$ sont choisis entre l'hydrogène, un groupe alkyle inférieur facultativement substitué, 1-adamantanoyle, acyle, phényle facultativement substitué ou (phényle facultativement substitué)-alkyle inférieur, et $R^4$ est défini comme étant un groupe alkyle inférieur facultativement substitué ; suivie

b) de la réaction du composé ainsi préparé de formule A avec la guanine facultativement substituée ; facultativement suivie

c) du traitement du mélange résultant avec un alcool alkylique inférieur ; et/ou

d) si $R^1$ et $R^3$ ne sont pas de l'hydrogène, d'un traitement du mélange résultant avec l'hydroxyde d'ammonium et l'eau.

**16.** Procédé suivant l'une quelconque des revendications 13 à 15, dans lequel on utilise la guanine non substituée.

**17.** Procédé suivant l'une quelconque des revendications 13 à 16, dans lequel $R^5$ est un groupe acyle.

**18.** Procédé suivant l'une quelconque des revendications 13 à 17, dans lequel $R^1$ et $R^3$ sont identiques.

**19.** Procédé suivant la revendication 18, dans lequel $R^1$ et $R^3$ représentent un groupe 1-adamantanoyle ou pivaloyle.

**Revendications pour l'Etat contractant suivant: AT**

**1.** Procédé de préparation d'un composé représenté par la formule A

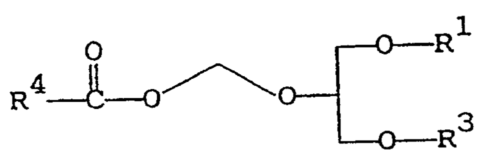

A

dans laquelle $R^1$ et $R^3$ sont choisis entre l'hydrogène, un groupe alkyle inférieur facultativement substitué, acyle, 1-adamantanoyle, phényle facultativement substitué ou (phényle facultativement substitué)-alkyle inférieur, et $R^4$ est un groupe alkyle inférieur facultativement substitué, comprenant :

a) la réaction d'un composé de formule D

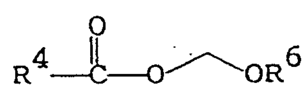

D

dans laquelle $R^4$ est tel que défini ci-dessus et $R^6$ est un groupe alkyle inférieur facultativement substitué, avec un composé de formule E

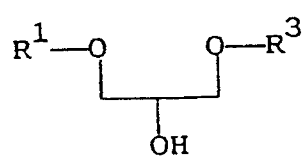

E

dans laquelle $R^1$ et $R^3$ sont tels que définis ci-dessus, en présence d'un acide protique sans apport de chaleur externe, pour former un composé de formule C

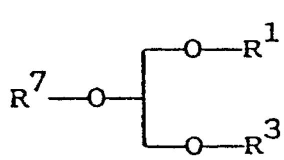

C

dans laquelle $R^1$ et $R^3$ sont tels que définis ci-dessus et $R^7$ est un groupe $R^4C(O)OCH_2$ ou $R^6OCH_2$ dans lequel $R^4$ et $R^6$ sont tels que définis ci-dessus ;

b) facultativement suivie, si $R^7$ est un groupe $R^6OCH_2$, de la réaction d'un composé représenté par

la formule B

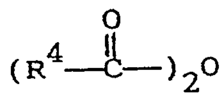 B

dans laquelle $R^4$ est choisi indépendamment dans le groupe tel que défini ci-dessus, avec un composé de formule C dans lequel $R^7$ est un groupe $R^6OCH_2$, en présence d'un acide de Lewis pendant environ 60 à 180 minutes à une température comprise entre 45 et 65 °C.

2. Procédé suivant la revendication 1, dans lequel $R^1$ et $R^3$ sont tous deux des groupes benzyle dans le composé représenté par la formule E.

3. Procédé suivant la revendication 1, dans lequel $R^4$ est un groupe méthyle dans le composé représenté par la formule B.

4. Procédé suivant la revendication 1, dans lequel le composé représenté par la formule E est un composé dans lequel $R^1$ et $R^3$ sont tous deux des groupes (1-adamantanoyle) et le composé représenté par B est l'anhydride acétique.

5. Procédé suivant la revendication 1, dans lequel le composé représenté par la formule E est un composé dans lequel $R^1$ et $R^3$ sont tous deux des groupes pivaloyle et le composé représenté par la formule B est l'anhydride acétique.

6. Procédé suivant la revendication 1, dans lequel on prépare un composé de formule A dans laquelle $R^1$ et $R^3$ sont choisis entre l'hydrogène, un groupe alkyle inférieur facultativement substitué, acyle, 1-adamantanoyle, phényle facultativement substitué ou (phényle facultativement substitué)-alkyle inférieur et $R^4$ est un groupe alkyle inférieur facultativement substitué ; excepté lorsque $R^4$ est un groupe méthyle auquel cas l'un au moins de $R^1$ et $R^3$ n'est pas un groupe acétyle, alkyle inférieur, phényle facultativement substitué ou (phényle facultativement substitué)-alkyle inférieur et que lorsque $R^4$ est un groupe éthyle, $R^1$ et $R^3$ ne peuvent pas être tous deux le même groupe alkyle inférieur.

7. Procédé suivant la revendication 6, dans lequel est préparé un composé dans la formule duquel $R^1$ et $R^3$ sont identiques.

8. Procédé suivant la revendication 7, dans lequel est préparé un composé dans lequel $R^4$ est un groupe méthyle.

9. Procédé suivant la revendication 8, dans lequel est préparé le 1,3-dipivaloyl-2-acétoxyméthoxyglycérol ou le 1,3-di-(1-adamantanoyl)-2-acétoxyméthoxyglycérol.

10. Procédé suivant la revendication 7, dans lequel est préparé un composé dans lequel $R^4$ est un groupe éthyle.

11. Procédé suivant la revendication 10, dans lequel est préparé le 1,3-dipivaloyl-2-propanoyloxyméthylglycérol ou le 1,3-di-(1-adamantanoyl)-2-propanoyloxyméthylglycérol.

12. Procédé suivant la revendication 1, dans lequel est préparé un composé de formule

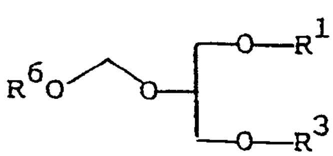 Formule F

dans laquelle $R^1$, $R^3$ et $R^6$ sont définis comme étant des groupes alkyle inférieurs éventuellement substitués, excepté que $R^1$ et $R^3$ ne peuvent pas être tous deux des groupes propyle.

**13.** Procédé de préparation de composés représentés par la formule

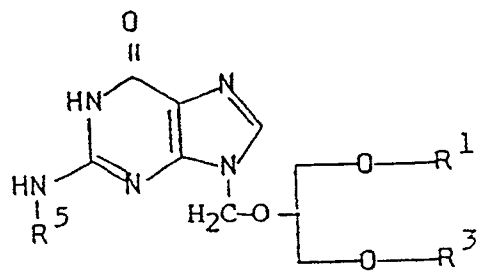

dans laquelle $R^1$ et $R^3$ sont choisis entre l'hydrogène, un groupe alkyle inférieur facultativement substitué, acyle, 1-adamantanoyle, phényle facultativement substitué ou (phényle facultativement substitué)-alkyle inférieur, et $R^5$ est un groupe acyle facultativement substitué ou l'hydrogène, comprenant :

a) la réaction de guanine facultativement substituée avec un excès d'hexa(alkyle inférieur)disilazane et un catalyseur, et avec un composé représenté par la formule :

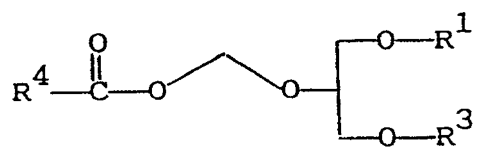

Formule A

dans laquelle $R^1$ et $R^3$ sont choisis entre l'hydrogène, un groupe alkyle inférieur facultativement substitué, 1-adamantanoyle, acyle, phényle facultativement substitué ou (phényle facultativement substitué)-alkyle inférieur, et $R^4$ est défini comme étant un groupe alkyle inférieur facultativement substitué ; suivie

b) du traitement du mélange résultant avec un alcool alkylique inférieur ; éventuellement suivi

c) si $R^1$ et $R^3$ ne sont pas de l'hydrogène, d'un traitement du mélange résultant avec l'hydroxyde d'ammonium et l'eau.

**14.** Procédé de préparation de composés représentés par la formule

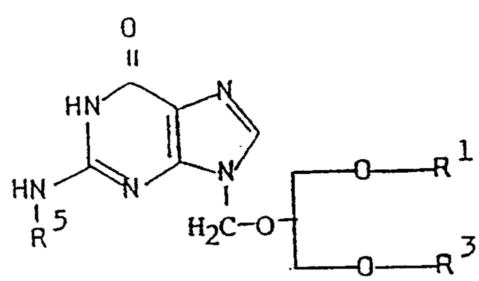

dans laquelle $R^1$ et $R^3$ représentent l'hydrogène, un groupe acyle ou le groupe 1-adamantanoyle et $R^5$ est un groupe acyle facultativement substitué ou l'hydrogène, comprenant

a) la réaction de guanine facultativement substituée en présence d'un catalyseur acide avec un composé représenté par la formule

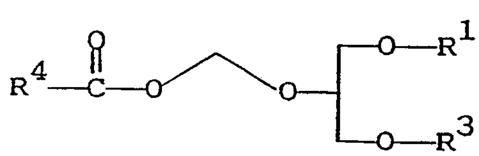

Formule A

dans laquelle $R^1$ et $R^3$ sont choisis entre des groupes acyle ou 1-adamantanoyle et $R^4$ est défini

comme étant un groupe alkyle inférieur facultativement substitué ; éventuellement suivie

b) du traitement du mélange résultant avec un alcool alkylique inférieur ; et/ou

c) si $R^1$ et $R^3$ ne sont pas de l'hydrogène, d'un traitement du mélange résultant avec l'hydroxyde d'ammonium et l'eau.

**15.** Procédé de préparation de composés représentés par la formule

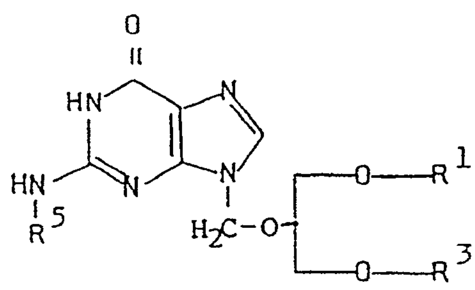

dans laquelle $R^1$ et $R^3$ sont choisis entre l'hydrogène, un groupe alkyle inférieur facultativement substitué, acyle, 1-adamantanoyle, phényle facultativement substitué ou (phényle facultativement substitué)-alkyle inférieur, et $R^5$ est un groupe acyle facultativement substitué ou l'hydrogène, comprenant :

a) la préparation selon la revendication 1 d'un composé de formule

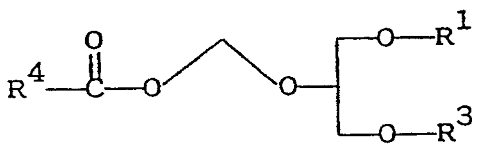

Formule A

dans laquelle $R^1$ et $R^3$ sont choisis entre l'hydrogène, un groupe alkyle inférieur facultativement substitué, 1-adamantanoyle, acyle, phényle facultativement substitué ou (phényle facultativement substitué)-alkyle inférieur, et $R^4$ est défini comme étant un groupe alkyle inférieur facultativement substitué ; suivie

b) de la réaction du composé ainsi préparé de formule A avec une guanine facultativement substituée ; facultativement suivie

c) du traitement du mélange résultant avec un alcool alkylique inférieur ; et/ou

d) si $R^1$ et $R^3$ ne sont pas de l'hydrogène, d'un traitement du mélange résultant avec l'hydroxyde d'ammonium et l'eau.

**16.** Procédé suivant l'une quelconque des revendications 13 à 15 , dans lequel la guanine non substituée est utilisée.

**17.** Procédé suivant l'une quelconque des revendications 13 à 16, dans lequel $R^5$ est un groupe acyle.

**18.** Procédé suivant l'une quelconque des revendications 13 à 17, dans lequel $R^1$ et $R^3$ sont identiques.

**19.** Procédé suivant la revendication 18, dans lequel $R^1$ et $R^3$ sont des groupes 1-adamantanoyle ou pivaloyle.

**Patentansprüche**

**Patentansprüche für folgende Vertragsstaaten: BE, FR, CH, DE, GB, IT, LI, LU, NL und SE**

**1.** Verfahren zur Herstellung einer Verbindung, die dargestellt wird durch Formel A

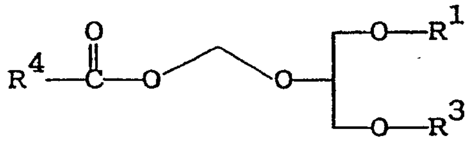

A

worin $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^4$ gegebenenfalls substituiertes Niederalkyl ist, umfassend:

a) das Umsetzen einer Verbindung der Formel D

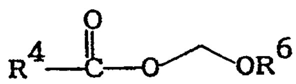

D

worin $R^4$ wie oben definiert ist und $R^6$ gegebenenfalls substituiertes Niederalkyl ist, mit einer Verbindung der Formel E

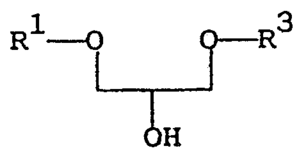

E

worin $R^1$ und $R^3$ wie oben definiert sind, in Gegenwart einer protischen Säure ohne äußere Hitze, um eine Verbindung der Formel C

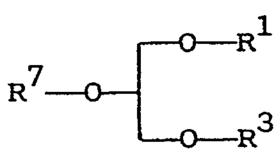

C

zu bilden, worin $R^1$ und $R^3$ wie oben definiert sind und $R^7$ $R^4C(O)OCH_2$ oder $R^6OCH_2$ ist, worin $R^4$ und $R^6$ wie oben definiert sind;

b) gegebenenfalls, falls $R^7$ $R^6OCH_2$ ist, gefolgt vom Umsetzen einer Verbindung, die dargestellt wird durch Formel B

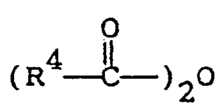

B

worin $R^4$ unabhängig ausgewählt ist aus der Gruppe, wie sie oben definiert wurde, mit einer Verbindung der Formel C, in der $R^7$ $R^6OCH_2$ ist, in Gegenwart einer Lewissäure, während ungefähr 60 bis 180 Minuten bei einer Temperatur zwischen 45° und 65° C.

**2.** Verfahren nach Anspruch 1, worin in der durch Formel E dargestellten Verbindung $R^1$ und $R^3$ beide Benzyl sind.

**3.** Verfahren nach Anspruch 1, worin in der durch Formel B dargestellten Verbindung $R^4$ Methyl ist.

**4.** Verfahren nach Anspruch 1, worin die durch Formel E dargestellte Verbindung eine Verbindung ist, worin $R^1$ und $R^3$ beide (1-Adamantanoyl)- sind, und die durch Formel B dargestellte Verbindung Essigsäureanhydrid ist.

5. Verfahren nach Anspruch 1, worin die durch Formel E dargestellte Verbindung eine Verbindung ist, worin $R^1$ und $R^3$ beide Pivaloyl sind, und die durch Formel B dargestellte Verbindung Essigsäureanhydrid ist.

6. Verbindung, die dargestellt wird durch die Formel

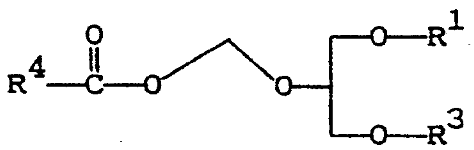

**Formel A**

worin $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^4$ gegebenenfalls substituiertes Niederalkyl ist; außer daß, wenn $R^4$ Methyl ist, mindestens eines von $R^1$ und $R^3$ nicht Acetyl, Niederalkyl, gegebenenfalls substituiertes Phenyl oder gegebenenfalls substituiertes Phenylniederalkyl ist; und daß, wenn $R^4$ Ethyl ist, $R^1$ und $R^3$ nicht beide die gleiche Niederalkylgruppe sein können.

7. Verbindung nach Anspruch 6, worin $R^1$ und $R^3$ gleich sind.

8. Verbindung nach Anspruch 7, worin $R^4$ Methyl ist.

9. Verbindung nach Anspruch 8, die 1,3-Dipivaloyl-2-acetoxymethoxyglycerin oder 1,3-Di-(1-adamantanoyl)-2-acetoxymethoxyglycerin ist.

10. Verbindung nach Anspruch 7, worin $R^4$ Ethyl ist.

11. Verbindung nach Anspruch 10, die 1,3-Dipivaloyl-2-propanoyloxymethylglycerin oder 1,3-Di-(1-adamantanoyl)-2-propanoyloxymethylglycerin ist.

12. Verbindung, die dargestellt wird durch die Formel

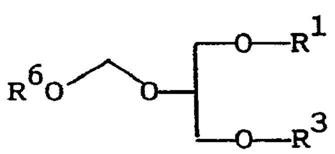

**Formel F**

worin $R^1$, $R^3$ und $R^6$ als gegebenenfalls substituiertes Niederalkyl definiert sind, außer daß $R^1$ und $R^3$ nicht beide Propyl sein können.

13. Verfahren zur Herstellung von Verbindungen, die dargestellt werden durch die Formel

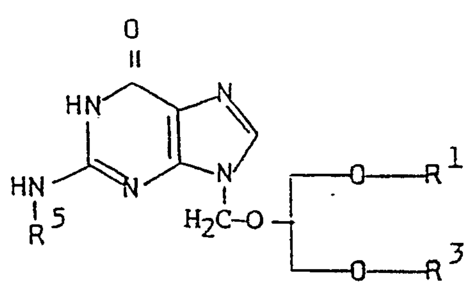

worin $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^5$ gegebenenfalls substituiertes Acyl oder Wasserstoff ist, umfassend:

a) das Umsetzen von gegebenenfalls substituiertem Guanin mit überschüssigem Hexa-Niederalkyldisilazan und einem Katalysator und mit einer Verbindung, die dargestellt wird durch die Formel:

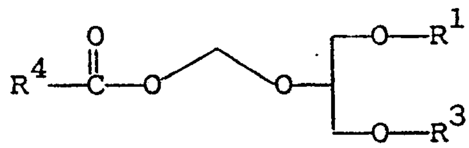

Formel A

worin $R^1$ und $R^3$ ausgewählt sind aus Wasserstoff, gegebenenfalls substituiertem Niederalkyl, 1-Adamantanoyl, Acyl, gegebenenfalls substituiertem Phenyl oder gegebenenfalls substituiertem Phenylniederalkyl und $R^4$ als gegebenenfalls substituiertes Niederalkyl definiert ist; gefolgt von

b) dem Behandeln der resultierenden Mischung mit einem Niederalkylalkohol; gegebenenfalls gefolgt von

c) falls $R^1$ und $R^3$ nicht Wasserstoff sind, dem Behandeln der resultierenden Mischung mit Ammoniumhydroxid und Wasser.

**14.** Verfahren zur Herstellung von Verbindungen, die dargestellt werden durch die Formel

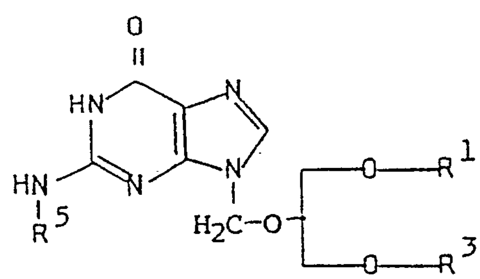

worin $R^1$ und $R^3$ Wasserstoff, Acyl oder 1-Adamantanoyl sind und $R^5$ gegebenenfalls substituiertes Acyl oder Wasserstoff ist, umfassend:

a) das Umsetzen von gegebenenfalls substituiertem Guanin in der Gegenwart eines sauren Katalysators mit einer Verbindung, die dargestellt wird durch die Formel:

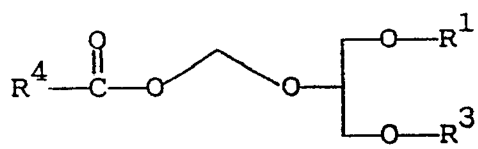

Formel A

worin $R^1$ und $R^3$ ausgewählt sind aus Acyl oder 1-Adamantanoyl und $R^4$ als gegebenenfalls substituiertes Niederalkyl definiert ist; gegebenenfalls gefolgt von

b) dem Behandeln der resultierenden Mischung mit einem Niederalkylalkohol; und/oder

c) falls $R^1$ und $R^3$ nicht Wasserstoff sind, dem Behandeln der resultierenden Mischung mit Ammoniumhydroxid und Wasser.

**15.** Verfahren zur Herstellung von Verbindungen, die dargestellt werden durch die Formel

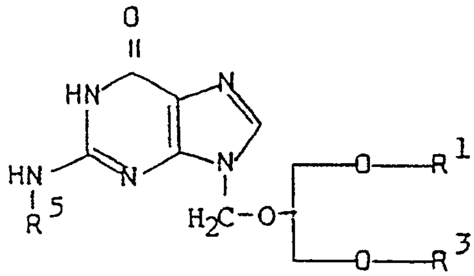

worin $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^5$ gegebenenfalls substituiertes Acyl oder Wasserstoff ist, umfassend:

a) die Herstellung nach Anspruch 1 einer Verbindung der Formel

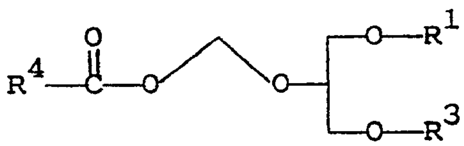

Formel A

worin $R^1$ und $R^3$ ausgewählt sind aus Wasserstoff, gegebenenfalls substituiertem Niederalkyl, 1-Adamantanoyl, Acyl, gegebenenfalls substituiertem Phenyl oder gegebenenfalls substituiertem Phenylniederalkyl und $R^4$ als gegebenenfalls substituiertes Niederalkyl definiert ist; gefolgt von

b) dem Umsetzen der so hergestellten Verbindung der Formel A mit gegebenenfalls substituiertem Guanin; gegebenenfalls gefolgt von

c) dem Behandeln der resultierenden Mischung mit einem Niederalkylalkohol; und/oder

d) falls $R^1$ und $R^3$ nicht Wasserstoff sind, dem Behandeln der resultierenden Mischung mit Ammoniumhydroxid und Wasser.

**16.** Verfahren nach irgendeinem der Ansprüche 13 bis 15, worin unsubstituiertes Guanin verwendet wird.

**17.** Verfahren nach irgendeinem der Ansprüche 13 bis 16, worin $R^5$ Acyl ist.

**18.** Verfahren nach irgendeinem der Ansprüche 13 bis 17, worin $R^1$ und $R^3$ gleich sind.

**19.** Verfahren nach Anspruch 18, worin $R^1$ und $R^3$ 1-Adamantanoyl oder Pivaloyl sind.

**Patentansprüche für folgenden Vertragsstaat: AT**

**1.** Verfahren zur Herstellung einer Verbindung, die dargestellt wird durch Formel A

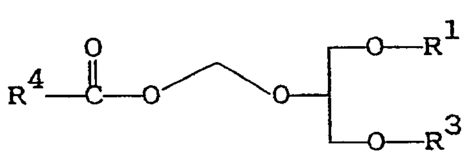

A

worin $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^4$ gegebenenfalls substituiertes Niederalkyl ist, umfassend:

a) das Umsetzen einer Verbindung der Formel D

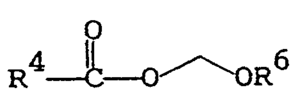

D

worin $R^4$ wie oben definiert ist und $R^6$ gegebenenfalls substituiertes Niederalkyl ist, mit einer Verbindung der Formel E

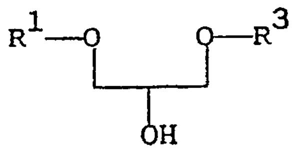

E

worin $R^1$ und $R^3$ wie oben definiert sind, in Gegenwart einer protischen Säure ohne äußere Hitze, um eine Verbindung der Formel C

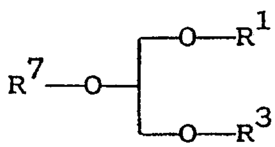

C

zu bilden, worin $R^1$ und $R^3$ wie oben definiert sind und $R^7$ $R^4C(O)OCH_2$ oder $R^6OCH_2$ ist, worin $R^4$ und $R^6$ wie oben definiert sind;
b) gegebenenfalls, falls $R^7$ $R^6OCH_2$ ist, gefolgt vom Umsetzen einer Verbindung, die dargestellt wird durch Formel B

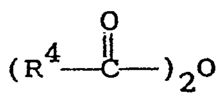

B

worin $R^4$ unabhängig ausgewählt ist aus der Gruppe, wie sie oben definiert wurde, mit einer Verbindung der Formel C, in der $R^7$ $R^6OCH_2$ ist, in Gegenwart einer Lewissäure, während ungefähr 60 bis 180 Minuten bei einer Temperatur zwischen 45° und 65° C.

2. Verfahren nach Anspruch 1, worin in der durch Formel E dargestellten Verbindung $R^1$ und $R^3$ beide Benzyl sind.

3. Verfahren nach Anspruch 1, worin in der durch Formel B dargestellten Verbindung $R^4$ Methyl ist.

4. Verfahren nach Anspruch 1, worin die durch Formel E dargestellte Verbindung eine Verbindung ist, worin $R^1$ und $R^3$ beide (1-Adamantanoyl)- sind, und die durch Formel B dargestellte Verbindung Essigsäureanhydrid ist.

5. Verfahren nach Anspruch 1, worin die durch Formel E dargestellte Verbindung eine Verbindung ist, worin $R^1$ und $R^3$ beide Pivaloyl sind, und die durch Formel B dargestellte Verbindung Essigsäureanhydrid ist.

6. Verfahren nach Anspruch 1, worin eine Verbindung der Formel A hergestellt wird, in der $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^4$ gegebenenfalls substituiertes Niederalkyl ist; außer daß, wenn $R^4$ Methyl ist, mindestens eines von $R^1$ und $R^3$ nicht Acetyl, Niederalkyl, gegebenenfalls substituiertes Phenyl oder gegebenenfalls substituiertes Phenylniederalkyl ist; und daß, wenn $R^4$ Ethyl ist, $R^1$ und $R^3$ nicht beide die gleiche Niederalkylgruppe sein können.

7. Verfahren nach Anspruch 6, worin eine Verbindung hergestellt wird, in der $R^1$ und $R^3$ gleich sind.

8. Verfahren nach Anspruch 7, worin eine Verbindung hergestellt wird, in der $R^4$ Methyl ist.

**9.** Verfahren nach Anspruch 8, worin 1,3-Dipivaloyl-2-acetoxymethoxyglycerin oder 1,3-Di-(1-adamantanoyl)-2-acetoxymethoxyglycerin hergestellt wird.

**10.** Verfahren nach Anspruch 7, worin eine Verbindung hergestellt wird, in der $R^4$ Ethyl ist.

**11.** Verfahren nach Anspruch 10, worin 1,3-Dipivaloyl-2-propanoyloxymethylglycerin oder 1,3-Di-(1-adamantanoyl)-2-propanoyloxymethylglycerin hergestellt wird.

**12.** Verfahren nach Anspruch 1, worin eine Verbindung der Formel F

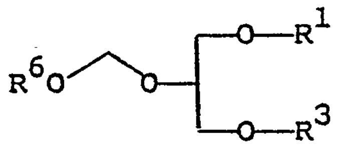

**Formel F**

hergestellt wird, in der $R^1$, $R^3$ und $R^6$ als gegebenenfalls substituiertes Niederalkyl definiert sind, außer daß $R^1$ und $R^3$ nicht beide Propyl sein können.

**13.** Verfahren zur Herstellung von Verbindungen, die dargestellt werden durch die Formel

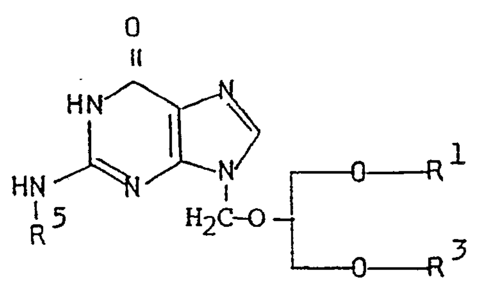

worin $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^5$ gegebenenfalls substituiertes Acyl oder Wasserstoff ist, umfassend:

a) das Umsetzen von gegebenenfalls substituiertem Guanin mit überschüssigem Hexa-Niederalkyldisilazan und einem Katalysator und mit einer Verbindung, die dargestellt wird durch die Formel:

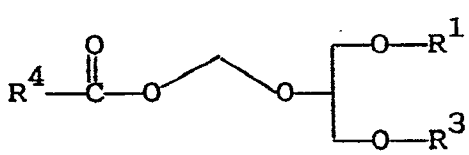

**Formel A**

worin $R^1$ und $R^3$ ausgewählt sind aus Wasserstoff, gegebenenfalls substituiertem Niederalkyl, 1-Adamantanoyl, Acyl, gegebenenfalls substituiertem Phenyl oder gegebenenfalls substituiertem Phenylniederalkyl und $R^4$ als gegebenenfalls substituiertes Niederalkyl definiert ist; gefolgt von

b) dem Behandeln der resultierenden Mischung mit einem Niederalkylalkohol; gegebenenfalls gefolgt von

c) falls $R^1$ und $R^3$ nicht Wasserstoff sind, dem Behandeln der resultierenden Mischung mit Ammoniumhydroxid und Wasser.

**14.** Verfahren zur Herstellung von Verbindungen, die dargestellt werden durch die Formel

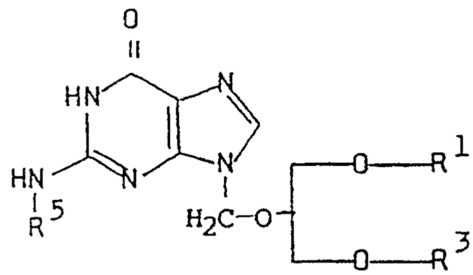

worin $R^1$ und $R^3$ Wasserstoff, Acyl oder 1-Adamantanoyl sind und $R^5$ gegebenenfalls substituiertes Acyl oder Wasserstoff ist, umfassend:

a) das Umsetzen von gegebenenfalls substituiertem Guanin in der Gegenwart eines sauren Katalysators mit einer Verbindung, die dargestellt wird durch die Formel:

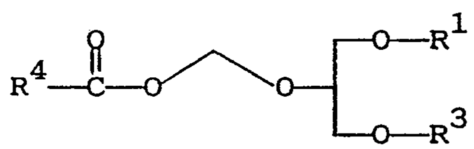

**Formel A**

worin $R^1$ und $R^3$ ausgewählt sind aus Acyl oder 1-Adamantanoyl und $R^4$ als gegebenenfalls substituiertes Niederalkyl definiert ist; gegebenenfalls gefolgt von

b) dem Behandeln der resultierenden Mischung mit einem Niederalkylalkohol; und/oder

c) falls $R^1$ und $R^3$ nicht Wasserstoff sind, dem Behandeln der resultierenden Mischung mit Ammoniumhydroxid und Wasser.

**15.** Verfahren zur Herstellung von Verbindungen, die dargestellt werden durch die Formel

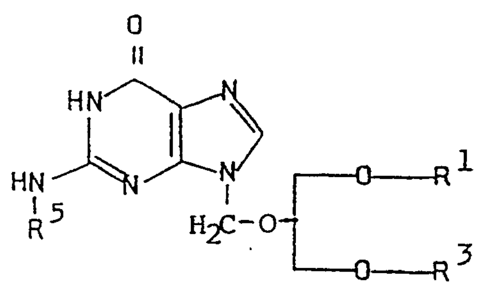

worin $R^1$ und $R^3$ ausgewählt sind aus Wasserstoff, gegebenenfalls substituiertem Niederalkyl, Acyl, 1-Adamantanoyl, gegebenenfalls substituiertem Phenyl oder gegebenenfalls substituiertem Phenylniederalkyl und $R^5$ gegebenenfalls substituiertes Acyl oder Wasserstoff ist, umfassend:

a) die Herstellung nach Anspruch 1 einer Verbindung der Formel

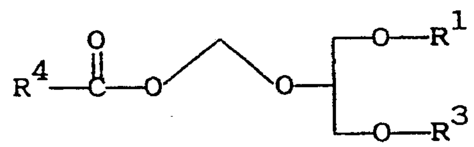

**Formel A**

worin $R^1$ und $R^3$ ausgewählt sind aus Wasserstoff, gegebenenfalls substituiertem Niederalkyl, 1-Adamantanoyl, Acyl, gegebenenfalls substituiertem Phenyl oder gegebenenfalls substituiertem Phenylniederalkyl und $R^4$ als gegebenenfalls substituiertes Niederalkyl definiert ist; gefolgt von

b) dem Umsetzen der so hergestellten Verbindung der Formel A mit gegebenenfalls substituiertem Guanin; gegebenenfalls gefolgt von

c) dem Behandeln der resultierenden Mischung mit einem Niederalkylalkohol; und/oder

d) falls $R^1$ und $R^3$ nicht Wasserstoff sind, dem Behandeln der resultierenden Mischung mit

Ammoniumhydroxid und Wasser.

**16.** Verfahren nach irgendeinem der Ansprüche 13 bis 15, worin unsubstituiertes Guanin verwendet wird.

**17.** Verfahren nach irgendeinem der Ansprüche 13 bis 16, worin $R^5$ Acyl ist.

**18.** Verfahren nach irgendeinem der Ansprüche 13 bis 17, worin $R^1$ und $R^3$ gleich sind.

**19.** Verfahren nach Anspruch 18, worin $R^1$ und $R^3$ 1-Adamantanoyl oder Pivaloyl sind.